# EUROPEAN PATENT SPECIFICATION

(11) **EP 1 224 323 B1**
(45) Date of publication and mention of the grant of the patent: **01.03.2006**
(21) Application number: 00922139.1
(22) Date of filing: 13.04.2000
(51) Int. Cl.: C12Q 1/68, A62D 3/00

(54) **NUCLEIC ACID FRAGMENTS FOR THE IDENTIFICATION OF DECHLORINATING BACTERIA**
NUKLEINSÄUREFRAGMENTE ZUR IDENTIFIZIERUNG VON DECHLORIERENDEN BAKTERIEN
FRAGMENTS D'ACIDE NUCLEIQUE DESTINES A L'IDENTIFICATION DE LA BACTERIE DE DECHLORATION

(30) Priority: 15.04.1999 US 129511 P
(43) Date of publication of application: 24.07.2002
(73) Proprietor: E.I. DU PONT DE NEMOURS AND COMPANY, Wilmington Delaware 19898 (US)
(72) Inventor: HENDRICKSON, Edwin, R., Hockessin, DE 19707 (US); EBERSOLE, Richard, C., Wilmington, DE 19810 (US)
(74) Representative: Grant, Anne Rosemary
(86) International application number: PCT/US2000/009883
(87) International publication number: WO 2000/063443

(56) References cited:
- EP-A- 0 864 542
- WO-A-98/49106
- FR-A- 2 733 754
- US-A- 5 540 838
- MAYMO-GATELL XAVIER ET AL: "Isolation of a bacterium that reductively dechlorinates tetrachloroethene to ethene." SCIENCE (WASHINGTON D C), vol. 276, no. 5318, 1997, pages 1568-1571, XP002179676 ISSN: 0036-8075 & DATABASE EMBL:DEAF4928 [Online] EMBL; 4 July 1997 (1997-07-04) MAYM0-GATELL XAVIER ET AL: retrieved from EMBL, accession no. AF004928 Database accession no. EMBL:DEAF4928
- HOLOMAN TRACEY R PULLIAM ET AL: "Characterization of a defined 2,3,5,6-tetrachlorobiphenyl-ortho-dechl orinating microbial community by comparative sequence analysis of genes coding for 16S rRNA." APPLIED AND ENVIRONMENTAL MICROBIOLOGY, vol. 64, no. 9, 1998, pages 3359-3367, XP002179677 ISSN: 0099-2240
- VON WINTZINGERODE FRIEDRICH ET AL: "Phylogenetic analysis of an anaerobic, trichlorobenzene-transforming microbial consortium." APPLIED AND ENVIRONMENTAL MICROBIOLOGY, vol. 65, no. 1, January 1999 (1999-01), pages 283-286, XP002179679 ISSN: 0099-2240
- LAMONTAGNE M G ET AL: "Identification and analysis of PCB dechlorinating anaerobic enrichments by amplification: Accuracy of community structure based on restriction analysis and partial sequencing of 16S rRNA genes." JOURNAL OF APPLIED MICROBIOLOGY, vol. 84, no. 6, June 1998 (1998-06), pages 1156-1162, XP001024984 ISSN: 1364-5072

### SEQUENCE LISTING

<110> E. I. DU PONT DE NEMOURS AND COMPANY
<120> NUCLEIC ACID FRAGMENTS FOR THE IDENTIFICATION OF DECHLORINATING BACTERIA
<130> BC1002 PCT
<140>
   <141>
<150> 60/129,511
   <151> 1999-04-15
<160> 33
<170> Microsoft Office 97
<210> 1
   <211> 24
   <212> DNA
   <213> Dehalococcoides ethenogenes
<400> 1
   attttctagc gagactgccc cgcg                                               24
<210> 2
   <211> 1212
   <212> DNA
   <213> Dehalococcoides ethenogenes
<400> 2
   gatgaacgct agcggcgtgc cttatgcatg caagtcgaac ggtcttaagc aattaagata   60
   gtggcaaacg ggtgagtaac gcgtaagtaa cctacctcta agtgggggat agcttcggga  120
   aactgaaggt aataccgcat gtgatgggct gacataagtc ggttcattaa agccgcaagg  180
   tgcttggtga ggggcttgcg tccgattagc tagttggtgg ggtaatggcc taccaaggct  240
   tcgatcggta gctggtctga gaggatgatc agccacactg ggactgagac acggcccaga  300
   ctcctacggg aggcagcagc aaggaatctt gggcaatggg cgaaagcctg acccagcaac  360
   gccgcgtgag ggatgaaggc tttcgggttg taaacctctt ttcacaggga agaataatga  420
   cggtacctgt ggaataagct tcggctaact acgtgccagc agccgcggta atacgtagga  480
   agcaagcgtt atccggattt attgggcgta aagtgagcgt aggtggtctt tcaagttgga  540
   tgtgaaattt cccggcttaa ccgggacgtg tcattcaata ctgttggact agagtacagc  600
   aggagaaaac ggaattcccg gtgtagtggt aaaatgcgta gatatcggga ggaacaccag  660
   aggcgaaggc ggttttctag gttgtcactg acactgaggc tcgaaagcgt ggggagcgaa  720
   cagaattaga tactctggta gtccacgcct taaactatgg acactaggta tagggagtat  780
   cgaccctctc tgtgccgaag ctaacgctyt aagtgtcccg cctggggagt acggtcgcaa  840
   ggctaaaact caaaggaatt gacgggggcc cttacaagca gcggagcgtg tggtttaatt  900
   cgatgctaca cgaagaacct taccaagatt tgacatgcat gaagtagtga accgaaaggg  960
   aaacgacctg ttaagtcagg agtttgcaca ggtgctgcat ggctgtcgtc agctcgtgcc 1020
   gtgaggtgtt tggttaagtc ctgcaacgag cgcaaccctt gttgctagtt aaattttcta 1080
   gcgagactgc cccgcgaaac ggggaggaag gtggggatga cgtcaagtca gcatggcctt 1140
   tatatcttgg gctacacaca cgctacaatg gacagaacaa taggttgcaa cagtgtgaac 1200
   tggagctaat cc                                                       1212
<210> 3
   <211> 1335
   <212> DNA
   <213> Dehalococcoides ethenogenes
<400> 3
   gatgaacgct agcggcgtgc cttatgcatg caagtcgaac ggtcttaagc aattaagata 60
   gtggcaaacg ggtgagtaac gcgtaagtaa cctacctcta agtgggggat agcttcggga 120
   aactgaaggt aataccgcat gtggtgggcc gacataagtt ggttcactaa agccgtaagg 180
   tgcttggtga ggggcttgcg tccgattagc tagttggtgg ggtaacggcc taccaaggct 240
   tcgatcggta gcttggtctg agaggatgat cagccacact gggactgaga cacggcccag 300
   actcctacgg gaggcagcag caaggaatct tgggcaatgg gcgaaagcct gacccagcaa 360
   cgccgcgtga gggatgaagg ctctcgggtt gtaaacctct tttcacaggg aagaataatg 420
   acggtacctg tggaataagc ttcggctaac tacgtgccag cagccgcggt aatacgtagg 480
   aagcaagcgt tatccggatt tattgggcgt aaagtgagcg taggtggtct ttcaagttgg 540
   atgtgaaatt tcccggctta accgggacgt gtcattcaat actgttggac tagagtacag 600
   caggagaaaa cggaattccc ggtgtagtgg taaaatgcgt agatatcggg aggaacacca 660
   gaggcgaagg cggttttcta ggttgtcact gacactgagg ctcgaaagcg tggggagcga 720
   acagaattag atactctggt agtccacgcc ttaaactatg gacactaagt atagggagta 780
   tcgaccctct ctgtgccgaa gctaacgctt taagtgtccc gcctggggag tacggtcgca 840
   aggctaaaac tcaaaggaat tgacgggggc ccgcacaagc agcggagcgt gtggtttaat 900
   tcgatgctac acgaagaacc ttaccaagat ttgacatgca tgaagtagtg aaccgaaagg 960
   gaaacgacct gttaagtcag gagtttgcac aggtgctgca tggctgtcgt cagctcgtgc 1020
   cgtgaggtgt ttggttaagt cctgcaacga gcgcaaccct tgttgctagt taaattttct 1080
   agcgagactg ccccgcgaaa cggggaggaa ggtggggatg acgtcaagtc agcatggcct 1140
   ttatatcttg ggctacacac acgctacaat ggacagaaca ataggttgca acagtgtgaa 1200
   ctggagctaa tcctcaaagc tgtcctcagt tcggattgca ggctgaaacc cgcctgcatg 1260
   aagttggagt tgctagtaac cgcatatcag caaggtgcgg tgaatacgtt ctcgggcctt 1320
   gtacacaccg cccgt 1335
<210> 4
   <211> 1212
   <212> DNA
   <213> Dehalococcoides ethenogenes
<400> 4
   gatgaacgct agcggcgtgc cttatgcatg caagtcgaac ggtcttaagc aattaagata 60
   gtggcgaacg ggtgagtaac gcgtaagtaa cctacctcta agtgggggat agcttcggga 120
   aactgaaggt aataccgcat gtggtgggcc gacatatgtt ggttcactaa agccgtaagg 180
   cgcttggtga ggggcttgcg tccgattagc tagttggtgg ggtaatggcc taccaaggct 240
   tcgatcggta gctggtctga gaggatgatc agccacactg ggactgagac acggcccaga 300
   ctcctacggg aggcagcagc aaggaatctt gggcaatggg cgaaagcctg acccagcaac 360
   gccgcgtgag ggatgaaggc tttcgggttg taaacctctt ttcataggga agaataatga 420
   cggtacctgt ggaataagct tcggctaact acgtgccagc agccgcggta atacgtagga 480
   agcaagcgtt atccggattt attgggcgta aagtgagcgt aggtggtctt tcaagttgga 540
   tgtgaaattt cccggcttaa ccgggacgag tcattcaata ctgttggact agagtacagc 600
   aggagaaaac ggaattcccg gtgtagtggt aaaatgcgta gatatcggga ggaacaccag 660
   aggcgaaggc ggttttctag gttgtcactg acactgaggc tcgaaagcgt ggggagcgaa 720
   cagaattaga tactctggta gtccacgcct taaactatgg acactaggta tagggagtat 780
   cgaccctctc tgtgccgaag ctaacgcttt aagtgtcccg cctggggagt acggtcgcaa 840
   ggctaaaact caaaggaatt gacgggggcc cgcacaagca gcggagcgtg tggtttaatt 900
   cgatgctaca cgaagaacct taccaagatt tgacatgcat gtagtagtga actgaaaggg 960
   gaacgacctg ttaagtcagg aacttgcaca ggtgctgcat ggctgtcgtc agctcgtgcc 1020
   gtgaggtgtt tggttaagtc ctgcaacgag cgcaaccctt gttgctagtt aaattttcta 1080
   gcgagactgc cccgcgaaac ggggaggaag gtggggatga cgtcaagtca gcatggcctt 1140
   tatatcttgg gctacacaca cgctacaatg gacagaacaa taggttgcaa cagtgtgaac 1200
   tggagctaat cc 1212
<210> 5
   <211> 1212
   <212> DNA
   <213> Dehalococcoides ethenogenes
<400> 5
   gatgaacgct agcggcgtgc cttatgcatg caagtcgaac ggtcttaagc aattaagata   60
   gtggcaaacg ggtgagtaac gcgtaagtaa cctacctcta agtgggggat agcttcggga  120
   aactgaaggt aataccgcat gtggtgggcc gacataagtt ggttcactaa agccgtaagg  180
   tgcttggtga ggggcttgcg tccgattagc tagttggtgg ggtaatggcc taccaaggct  240
   tcgatcggta gctggtctga gaggatgatc agccacactg ggactgagac acggcccaga  300
   ctcctacggg aggcagcagc aaggaatctt gggcaatggg cgaaagcctg acccagcaac  360
   gccgcgtgag ggatgaaggc tttcgggttg taaacctctt ttcacaggga agaataatga  420
   cggtacctgt ggaataagct tcggctaact acgtgccagc agccgcggta atacgtagga  480
   agcaagcgtt atccggattt attgggcgta aagtgagcgt aggtggtctt tcaagttgga  540
   tgtgaaattt cccggcttaa ccgggacgtg tcattcaata ctgttggact agagtacagc  600
   aggagaaaac ggaattcccg gtgtagtggt aaaatgcgta gatatcggga ggaacaccag  660
   aggcgaaggc ggttttctag gttgtcactg acactgaggc tcgaaagcgt ggggagcgaa  720
   cagaattaga tactctggta gtccacgcct taaactatgg acactaggta tagggagtat  780
   cgaccctctc tgtgccgaag ctaacgcttt aagtgtcccg cctggggagt acggtcgcaa  840
   ggctaaaact caaaggaatt gacgggggcc cgcacaagca gcggagcgtg tggtttaatt  900
   cgatgctaca cgaagaacct taccaagatt tgacatgcat gaagtagtga accgaaaggg  960
   aaacgacctg ttaagtcagg agtttgcaca ggtgctgcat ggctgtcgtc agctcgtgcc 1020
   gtgaggtgtt tggttaagtc ctgcaacgag cgcaaccctt gttgctagtt aaattttcta 1080
   gcgagactgc cccgcgaaac ggggaggaag gtggggatga cgtcaagtca gcatggcctt 1140
   tatatcttgg gctacacaca cgctacaatg gacagaacaa taggttgcaa cagtgtgaac 1200
   tggagctaat cc                                                    1212
<210> 6
   <211> 1212
   <212> DNA
   <213> Dehalococcoides ethenogenes
<400> 6
   gatgaacgct agcggcgtgc cttatgcatg caagtcgaac ggtcttaagc aattaagata   60
   gtggcaaacg ggtgagtaac gcgtaagtaa cctacctcta agtgggggat agcttcggga  120
   aactgaaggt aataccgcat gtggtgggcc gacataagtt ggttcactaa agccgtaagg  180
   tgcttggtga ggggcttgcg tccgattagc tagttggtgg ggtaacggcc taccaaggct  240
   tcgatcggta gctggtctga gaggatgatc agccacactg ggactgagac acggcccaga  300
   ctcctacggg aggcagcagc aaggaatctt gggcaatggg cgaaagcctg acccagcaac  360
   gccgcgtgag ggatgaaggc tctcgggttg taaacctctt ttcacaggga agaataatga  420
   cggtacctgt ggaataagct tcggctaact acgtgccagc agccgcggta atacgtagga  480
   agcaagcgtt atccggattt attgggcgta aagtgagcgt aggtggtctt tcaagttgga  540
   tgtgaaattt cccggcttaa ccgggacgtg tcattcaata ctgttggact agagtacagc  600
   aggagaaaac ggaattcccg gtgtagtggt aaaatgcgta gatatcggga ggaacaccag  660
   aggcgaaggc ggttttctag gttgtcactg acactgaggc tcgaaagcgt ggggagcgaa  720
   cagaattaga tactctggta gtccacgcct taaactatgg acactaggta tagggagtat  780
   cgaccctctc tgtgccgaag ctaacgcttt aagtgtcccg cctggggagt acggtcgcaa  840
   ggctaaaact caaaggaatt gacgggggcc cgcacaagca gcggagcgtg tggtttaatt  900
   cgatgctaca cgaagaacct taccaagatt tgacatgcat gaagtagtga accgaaaggg  960
   aaacgacctg ttaagtcagg agtttgcaca ggtgctgcat ggctgtcgtc agctcgtgcc 1020
   gtgaggtgtt tggttaagtc ctgcaacgag cgcaaccctt gttgctagtt aaattttcta 1080
   gcgagactgc cccgcgaaac ggggaggaag gtggggatga cgtcaagtca gcatggcctt 1140
   tatatcttgg gctacacaca cgctacaatg gacagaacaa taggttgcaa cagtgtgaac 1200
   tggagctaat cc                                                    1212
<210> 7
   <211> 1443
   <212> DNA
   <213> Dehalococcoides ethenogenes
<400> 7
   gatgaacgct agcggcgtgc cttatgcatg caagtcgaac ggtcttaagc aattaagata   60
   gtggcaaacg ggtgagtaac gcgtaagtaa cctacctcta agtgggggat agcttcggga  120
   aactgaaggt aataccgcat gtgatgggct gacataagtc ggttcattaa agccgcaagg  180
   tgcttggtga ggggcttgcg tccgattagc tagttggtgg ggtaatggtc taccaaggct  240
   tcgatcggta gctggtctga gaggatgatc agccacactg ggactgagac acgggccaga  300
   ctcctacggg aggcagcagc aaggaatctt gggcaatggg cgaaagcctg acccagcaac  360
   gccgcgtgag ggatgaaggc tttcgggttg taaacctctt ttcacaggga agaataatga  420
   cggtacctgt ggaataagct tcggctaact acgtgccagc agccgcggta atacgtaggg  480
   aagcaagcgt tatccggatt tattgggcgt aaagtgagcg taggtggtct ttcaagttgg  540
   atgtgaaatt tcccggctta accgggacgt gtcattcaat actgttggac tagagtacag  600
   caggagaaaa cggaattccc ggtgtagtgg taaaatgcgt agatatcggg aggaacacca  660
   gaggcgaagg cggttttcta ggttgtcact gacactgagg ctcgaaagcg tggggagcga  720
   acagaattag atactctggt agtccacgcc ttaaactatg gacactaggt atagggagta  780
   tcgaccctct ctgtgccgaa gctaacgctt taagtgtccc gcctggggag tacggtcgca  840
   aggctaaaac tcaaaggaat tgacgggggc ccgcacaagc agcggagcgt gtggtttaat  900
   tcgatgctac acgaagaact taccaagatt tgacatgcat gaagtagtga accgaaaggg  960
   aaacgacctg ttaagtcagg agtttgcaca ggtgctgcat ggctgtcgtc agctcgtgcc 1020
   gtgaggtgtt gggttaagtc ctgcaacgag cgcaaccttg ttgctagtta aattttctag 1080
   cgagactagc gagactgccc cgcgaaacgg ggaggaaggt ggggatgacg tcaagtcagc 1140
   atggccttta tatcttgggc tacacacacg ctacaatgga cagaacaata ggttgcaaca 1200
   gtgtgaactg gagctaatcc ccaaagctgt cctcagttcg gattgcaggc tgaaacccgc 1260
   ctgcatgaag ttggagttgc tagtaaccgc atatcagcaa ggtgcggtga atacgttctc 1320
   gggccttgta cacaccgccc gtcacgtcat ganagccggt aacacttgaa gtcgatgtgc 1380
   caaccgcaag gaggcagtcg ccgagggtgg gactggtaat tgggacgaag tcgtaacaag 1440
   gta                                                               1443
<210> 8
   <211> 47
   <212> DNA
   <213> Artificial Sequence
<220>
   <223> Description of Artificial Sequence: CONSENSUS
<220>
   <221> unsure
   <222> (5)
   <223> R=A/G
<220>
   <221> unsure
   <222> (11)
   <223> Y=C/T
<220>
   <221> unsure
   <222> (18)
   <223> W=A/T
<220>
   <221> unsure
   <222> (21)
   <223> Y=C/T
<220>
   <221> unsure
   <222> (28)
   <223> Y=T/C
<220>
   <221> unsure
   <222> (37)
   <223> Y=T/C
<220>
   <221> unsure
   <222> (42)
   <223> Y=C/T
<400> 8
   tgtgrtgggc ygacatawgt yggttcayta aagccgyaag gygcttg 47
<210> 9
   <211> 20
   <212> DNA
   <213> Artificial Sequence
<220>
   <223> Description of Artificial Sequence: PRIMER
<400> 9
   aagtcgaacg gtcttaagca 20
<210> 10
   <211> 20
   <212> DNA
   <213> Artificial Sequence
<220>
   <223> Description of Artificial Sequence: PRIMER
<400> 10
   cgtcattatt cttccctgtg 20
<210> 11
   <211> 21
   <212> DNA
   <213> Artificial Sequence
<220>
   <223> Description of Artificial Sequence: PRIMER
<400> 11
   gggaaacgac ctgttaagtc a 21
<210> 12
   <211> 22
   <212> DNA
   <213> Artificial Sequence
<220>
   <223> Description of Artificial Sequence: PRIMER
<400> 12
   ggattagctc cagttcacac tg 22
<210> 13
   <211> 20
   <212> DNA
   <213> Artificial Sequence
<220>
   <223> Description of Artificial Sequence: PRIMER
<400> 13
   aaatttaact agcaacaagg 20
<210> 14
   <211> 19
   <212> DNA
   <213> Artificial Sequence
<220>
   <223> Description of Artificial Sequence: PRIMER
<400> 14
   ggagtatcga ccctctctg 19
<210> 15
   <211> 18
   <212> DNA
   <213> Artificial Sequence
<220>
   <223> Description of Artificial Sequence: PRIMER
<400> 15
   gggagtatcg accctctc 18
<210> 16
   <211> 18
   <212> DNA
   <213> Artificial Sequence
<220>
   <223> Description of Artificial Sequence: PRIMER
<400> 16
   agtgaaccga aagggaaa 18
<210> 17
   <211> 21
   <212> DNA
   <213> Artificial Sequence
<220>
   <223> Description of Artificial Sequence: PRIMER
<400> 17
   gggttgtaaa cctcttttca c 21
<210> 18
   <211> 20
   <212> DNA
   <213> Artificial Sequence
<220>
   <223> Description of Artificial Sequence: PRIMER
<400> 18
   gttagcttcg gcacagagag 20
<210> 19
   <211> 20
   <212> DNA
   <213> Artificial Sequence
<220>
   <223> Description of Artificial Sequence: PRIMER
<400> 19
   tcagtgacaa cctagaaaac 20
<210> 20
   <211> 17
   <212> DNA
   <213> Artificial Sequence
<220>
   <223> Description of Artificial Sequence: PRIMER
<400> 20
   gatgaacgct agcggcg 17
<210> 21
   <211> 18
   <212> DNA
   <213> Artificial Sequence
<220>
   <223> Description of Artificial Sequence: PRIMER
<400> 21
   gtgccttatg catgcaag 18
<210> 22
   <211> 21
   <212> DNA
   <213> Artificial Sequence
<220>
   <223> Description of Artificial Sequence: PRIMER
<400> 22
   aataggttgc aacagtgtga a 21
<210> 23
   <211> 22
   <212> DNA
   <213> Artificial Sequence
<220>
   <223> Description of Artificial Sequence: PRIMER
<400> 23
   aatggacaga acaataggtt gc 22
<210> 24
   <211> 21
   <212> DNA
   <213> Artificial Sequence
<220>
   <223> Description of Artificial Sequence: PRIMER
<400> 24
   ggcacatcga cttcaagtgt t 21
<210> 25
   <211> 21
   <212> DNA
   <213> Artificial Sequence
<220>
   <223> Description of Artificial Sequence: PRIMER
<400> 25
   ggcacatcga cttcaagtgt t 21
<210> 26
   <211> 20
   <212> DNA
   <213> Artificial Sequence
<220>
   <223> Description of Artificial Sequence: PRIMER
<400> 26
   taaccgggac gngtcattca 20
<210> 27
   <211> 19
   <212> DNA
   <213> Artificial Sequence
<220>
   <223> Description of Artificial Sequence: PRIMER
<400> 27
   gagtacagca ggagaaaac 19
<210> 28
   <211> 21
   <212> DNA
   <213> Artificial Sequence
<220>
   <223> Description of Artificial Sequence: PRIMER
<400> 28
   cctccttgcg gttggcacat c 21
<210> 29
   <211> 19
   <212> DNA
   <213> Artificial Sequence
<220>
   <223> Description of Artificial Sequence: PRIMER
<400> 29
   ggcagtctcg ctagaaaat 19
   <210> 30
   <211> 51
   <212> DNA
   <213> Artificial Sequence
<220>
   <223> Description of Artificial Sequence: CONSENSUS
<220>
   <221> unsure
   <222> (3)
   <223> W=A/T
<220>
   <221> unsure
   <222> (14)
   <223> M=A/C
<220>
   <221> unsure
   <222> (22)
   <223> R=A/G
<220>
   <221> unsure
   <222> (43)
   <223> R=A/G
<220>
   <221> unsure
   <222> (44)
   <223> M=A/C
<400> 30
   tgwagtagtg aacmgaaagg graacgacct gttaagtcag garmttgcac a 51
   <210> 31
   <211> 18
   <212> DNA
   <213> Dehalococcoides ethenogenes
<400> 31
   attttctacg cgagactg 18
   <210> 32
   <211> 27
   <212> DNA
   <213> Dehalococcoides ethenogenes
<400> 32
   attttctacg cgagactagc gagactg 27
<210> 33
   <211> 1542
   <212> DNA
   <213> E. COLI
<400> 33
   aaattgaaga gtttgatcat ggctcagatt gaacgctggc ggcaggccta acacatgcaa   60
   gtcgaacggt aacaggaaga agcttgcttc tttgctgacg agtggcggac gggtgagtaa  120
   tgtctgggaa actgcctgat ggagggggat aactactgga aacggtagct aataccgcat  180
   aacgtcgcaa gaccaaagag ggggaccttc gggcctcttg ccatcggatg tgcccagatg  240
   ggattagcta gtaggtgggg taacggctca cctaggcgac gatccctagc tggtctgaga  300
   ggatgaccag ccacactgga actgagacac ggtccagact cctacgggag gcagcagtgg  360
   ggaatattgc acaatgggcg caagcctgat gcagccatgc cgcgtgtatg aagaaggcct  420
   tcgggttgta aagtactttc agcggggagg aagggagtaa agttaatacc tttgctcatt  480
   gacgttaccc gcagaagaag caccggctaa ctccgtgcca gcagccgcgg taatacggag  540
   ggtgcaagcg ttaatcggaa ttactgggcg taaagcgcac gcaggcggtt tgttaagtca  600
   gatgtgaaat ccccgggctc aacctgggaa ctgcatctga tactggcaag cttgagtctc  660
   gtagaggggg gtagaattcc aggtgtagcg gtgaaatgcg tagagatctg gaggaatacc  720
   ggtggcgaag gcggccccct ggacgaagac tgacgctcag gtgcgaaagc gtggggagca  780
   aacaggatta gataccctgg tagtccacgc cgtaaacgat gtcgacttgg aggttgtgcc  840
   cttgaggcgt ggcttccgga gctaacgcgt taagtcgacc gcctggggag tacggccgca  900
   aggttaaaac tcaaatgaat tgacgggggc ccgcacaagc ggtggagcat gtggtttaat  960
   tcgatgcaac gcgaagaacc ttacctggtc ttgacatcca cggaagtttt cagagatgag 1020
   aatgtgcctt cgggaaccgt gagacaggtg ctgcatggct gtcgtcagct cgtgttgtga 1080
   aatgttgggt taagtcccgc aacgagcgca acccttatcc tttgttgcca gcggtccggc 1140
   cgggaactca aaggagactg ccagtgataa actggaggaa ggtggggatg acgtcaagtc 1200
   atcatggccc ttacgaccag ggctacacac gtgctacaat ggcgcataca aagagaagcg 1260
   acctcgcgag agcaagcgga cctcataaag tgcgtcgtag tccggattgg agtctgcaac 1320
   tcgactccat gaagtcggaa tcgctagtaa tcgtggatca gaatgccacg gtgaatacgt 1380
   tcccgggcct tgtacacacc gcccgtcaca ccatgggagt gggttgcaaa agaagtaggt 1440
   agcttaacct tcgggagggc gcttaccact ttgtgattca tgactggggt gaagtcgtaa 1500
   caaggtaacc gtaggggaac ctgcggttgg atcacctcct ta                    1542

## Description

### FIELD OF THE INVENTION

The invention relates to the field of molecular biology and microbiology. More specifically, 16S rRNA regions of been identified and isolated from *Dehalococcoides ethenogenes* that enable the identification of dechlorinating bacterial strains. Probes and primers corresponding to the unique regions have been constructed to enable the rapid identification of the dechlorinators.

### BACKGROUND

Groundwater pollution by halogenated, and particularly chlorinated solvents is a worldwide problem associated primarily with industrial sites where mishandling or improper disposal has brought these solvents in contact with the soil. The most common and problematic compounds are the chlorinated ethylenes (ethenes) such as tetra- tri- or di-chloroethylene. Carbon tetrachloride, chloroform and methylene chloride are also pervasive pollutants. The reasons for concern are basically threefold. First, most of these solvents are sparingly soluble in water and have the tendency to stick to soil particles. This results in tenacious underground plumes of solvent which cannot readily be removed by standard pump and treat technology (Biswas, N., et al., *Water Environ. Res.* 64, 170, 10, 1 (1992); Hutter, G. M., et. al., *Water Environ. Res.* 64, 69, (1992)). Second, the toxicology of many chlorinated solvents suggests that these compounds may be carcinogenic and damaging to specific organs such as the liver and kidneys (Price, P. S., Memo of the U.S. Environmental Protection Agency, Office of Water, Washington, D.C.(1985); Vogel, T. M., *Environ. Sci. Technol.,* 21, 722, (1987)). Finally, under conditions found in many aquifers and subsurface environments, chlorinated ethylenes and methanes are very slow to be degraded biologically. The result of these factors is that chlorinated solvents are long-lived potentially hazardous groundwater pollutants.

Currently there are two approaches to in situ removal of organohalogen pollutants. The first approach is the standard "pump and treat" method where groundwater is pumped to the surface for physical stripping of the contaminant from the water. For chlorinated solvents this is more of a containment method than a remediation technology although given sufficient time (typically decades to centuries) this method may capture most of the pollutant. The other approach is biological in nature and utilizes microorganisms for the enzymatic transformation of the halogenated organics. The biological approach may utilize microorganisms indigenous to a particular site where the remediation process consists primarily of making additions to the contaminated site that enhance the growth of the desired microorganism. Alternatively, nonindigenous microorganisms may be introduced to a contaminated site with the necessary amendments needed for growth.

A number of organisms are known to dechlorinate persistent chlorinated pohutants. For example, *Dehalobacter restrictus* and *Dehalospirillium multivorans,* have been shown to partially dechlorinate chlorinated ethenes (Kochian et al., *Plant Mol. Biol.* 46:237 (1995); Delhaize et al., *Plant Physiol.* 107:315 (1995)). Additionally, WO 9849106 teaches the use of a hydrogenotrophic bacterium (*Dehalococcoides ethenogenes*) in a device for the decontamination of wastewater containing chlorinated compounds. Similarly, *Dehalococcoides ethenogenes* has been shown to effect the complete dechlorination of tetrachloroethene and trichloroethene to ethene [Freedman et al-, *Appl. Environ. Microbiol.* 55:2144 (1989)] and Maymó-Gatell et al. *(Science,* 176:1568 (1997)) have isolated a *D*. *ethenogenes* strain that is capable of respiratory reductive dechlorination of tetrachloroethene directly to ethene with hydrogen as an electron donor. Analysis of the 16S rRNA of the Maymó-Gatell organism revealed a unique profile that may be used to identify organisms of similar reductive capabilities.

The first step in utilizing the dechlorinating properties of the above identified organisms is rapid and accurate identification. One method of identification involves the use of DNA probes (see for example in WO 89/06704, U.S. Patent No. 4,851,330, and U.S. Patent No. 5,574,145). Many such probes derive from the observation (see Woese, *Scientific American* 244 (6) 1981 for review) that parts of the 16S and 23s ribosomal RNA (rRNA) sequences vary in different species. This information was used initially for phylogenetic analyses but it has more recently been used for DNA probe-based methods for the identification of organisms. The utility of such a method is based on the conservation of nucleic acid sequence within the rRNA sequences.

Each of the cells of all life forms, except viruses, contain ribosomes and therefore ribosomal RNA. A ribosome contains three separate single strand RNA molecules, namely, a large molecule, a medium sized molecule, and a small molecule. The two larger rRNA molecules vary in size in different organisms. Ribosomal RNA is a direct gene product and is coded for by the rRNA gene. This DNA sequence is used as a template to synthesize rRNA molecules. A separate gene exists for each of the ribosomal RNA subunits. Multiple rRNA genes exist in most organisms, many higher organisms containing both nuclear and mitochondrial rRNA genes. Numerous ribosomes are present in all cells of all life forms. About 85-90 percent of the total RNA in a typical cell is rRNA. A bacteria such as *E. coli* contains about 10⁴ ribosomes per cell. Much of the sequences in rRNA highly conserved across broad evolutionary boundaries, however, certain regions are highly variable and may be used to make fine distinctions between species, sub-species and strains (U.S. Patent No. SS67587). 16srDNA has been used to detect and isolate dechlorinating bacteria. For example, Pulliam et al., (*Applied and Environmental Microbiology,* 64, 3359, (1998)), has characterized a dechlorinating bacterial population on the basis of 16srDNA; Friedrich et al (*Applied and Environmental Microbiology,* 65, 283, (1999)) has isolated a number of trichlorobenzene degraders using small-subunit rRNA genes; and Horvais A, (FR 2733754) has isolated 16s RNA fragments from Clavibacter michiganensis (a dechlorinator)

The problem to be overcome therefore is to identify a unique 16S rDNA sequence in a bacteria capable of dechlorination of persistent chlorinated compounds for the identification and ultimate enhancement of that bacteria to remediated a contaminated site. Applicants have solved the state problem by providing a set of nucleic acid sequences that are unique to various strains of *Dehalococcoides ethenogenes.*

### SUMMARY OF THE INVENTION

The present invention provides an isolated 16S rDNA sequence indicative of a dechlorinating bacterial strain selected from the group consisting of: (a) SEQ ID NO:8 and SEQ ID NO:30; and (b) an isolated nucleic acid molecule that is completely complementary to (a).

The invention additionally provides an isolated bacterial strain comprising any one of the sequences of the instant invention as set forth in SEQ ID NO:8 and SEQ ID NO:30, wherein said strain has the ability to dechlorinate chlorinated compounds.

The invention further provides a method for identifying a dechlorinating bacterial strain comprising: (i) extracting genomic DNA from a cell suspected of being able to dechlorinate chlorinated compounds; (ii) probing the extracted genomic DNA with a probe derived from any one of the sequences of SEQ ID NO: 8 and SEQ ID NO:30, under suitable hybridization conditions, wherein the identification of a hybridizable nucleic acid fragment confirms the presence of a bacteria capable of dechlorinating chlorinated compounds.

Similarly the invention provides a method for identifying a dechlorinating bacterial strain comprising (i) extracting genomic DNA from a cell suspected of being able to dechlorinate chlorinated compounds; and (ii) amplifying the extracted genomic DNA with an oligonucleotide primer corresponding to a portion of any one of the sequences instant invention as set forth in SEQ ID NO:8 and SEQ ID NO:30, such that amplification products are generated wherein the presence of amplification products confirms the presence of a dechlorinating bacterial strain.

The invention additionally provides a method for the dechlorination of chlorinated compounds comprising contacting a chlorinated compound with an isolated bacterial strain comprising any one of the DNA fragments as set forth in SEQ ID NO:8 and SEQ ID NO:30 under conditions suitable for dechlorination to occur.

### BRIEF DESCRIPTION OF THE DRAWINGS AND SEQUENCE LISTING

Figure 1 is an alignment of 16S rDNA sequence profile from *Dehalococcoides ethenogenes* DHE-195 as disclosed in Maymó-Gatell et al., *Science,* 176:1568 (1997), as compared with profiles generated for organisms isolated from a number of wastewater treatment sites.
Figure 2 is a comparison of the instant dechlorinating 16S rDNA profiles with a 16S rDNA profile from *E*. *coli.*
Figure 3 is a graph illustrating the ability of a soil microcosm or culture developed from certain soils taken from a chloroethene contaminated site to dechlorinate trichloroethylene or perchloroethylene.
Figure 4 is an image of an electrophoresis gel used to detect PCR products in a test of soils contaminated with chloroethenes using two sets of the primers described herein. The Sequence Descriptions contain the one letter code for nucleotide sequence characters and the three letter codes for amino acids as defined in conformity with the IUPAC-IYUB standards described in *Nucleic Acids Research 13*:3021-3030 (1985) and in the *Biochemical Journal 219* (*No. 2)*:345-373 (1984).

SEQ ID NO:1 is a unique region of the *Dehalococcoides ethenogenes* 16S rDNA profile which is linked to dechlorinating activity.

SEQ ID NO:2 is the 16S rDNA profile of *Dehalococcoides ethenogenes* DHE-PL, isolated from soil surrounding in industrial site.

SEQ ID NO:3 is the 16S rDNA profile of *Dehalococcoides ethenogenes* DHE-STF, isolated from soil surrounding in industrial site.

SEQ ID NO:4 is the 16S rDNA profile of *Dehalococcoides ethenogenes* DHE-DAB, isolated from soil surrounding in industrial site.

SEQ ID NO:5 is the 16S rDNA profile of *Dehalococcoides ethenogenes* DHE-PIN, isolated from soil surrounding in industrial site.

SEQ ID NO:6 is the 16S rDNA profile of *Dehalococcoides ethenogenes* DHE-DLL, isolated from soil surrounding in industrial site.

SEQ ID NO:7 is the 16S rDNA profile of *Dehalococcoides ethenogenes* DHE-195 as reported in Maymó-Gatell et al. (*Science,* 176:1568 (1997)), Genbank AF004928.

SEQ ID NO:8 is the consensus sequence derived from DHE-PL, DHE-STF, DHE-DAB, DHE-PIN, and DHE-DLL at bases E180-E226.

SEQ ID NO:9-29 are primers derived from the 16S rDNA profile, useful in the identification of dechlorinating bacteria.

SEQ ID NO:30 is the consensus sequence derived from DHE-PL, DHE-STF, DHE-DAB, DHE-PIN, and DHE-DLL at bases E1001-E1047.

SEQ ID NO:31 is the base sequence in the region of the consensus 16S rDNA profile from where the diagnostic sequence is derived.

SEQ ID NO:32 is the base sequence in the region of the DHE-195 16S rDNA profile from where the diagnostic sequence is derived.

SEQ ID NO:33 is the *E. coli* reference 16S rDNA sequence.

### DETAILED DESCRIPTION OF THE INVENTION

The present invention provides unique 16S rDNA sequence profiles derived from *Dehalococcoides ethenogenes* (DHE). *D. ethenogenes* is known for its ability to degrade persistent chlorinated pollutants. The instant sequence profiles may be used to identify and sub-type bacteria with similar metabolic pathways. One sequence (ATTTTCTAGCGAGACTGCCCCGCG, SEQ ID NO:1), beginning at base E 1146, has been identified in all DHE's isolated from contaminated soils and is strongly linked to the ability of these organisms to degrade chlorinated organics. Similarly, a stretch of nucleic acids ranging between E180 and E226, corresponding to SEQ ID NO:8 may be used to identify dechlorinators as well as for genetic sub-typing of species.

In this disclosure, a number of terms and abbreviations are used. The following definitions are provided.

The term *"Dehalococcoides ethenogenes"* will be abbreviated "DHE".

The term "DHE-195" will refer to the strains of *Dehalococcoides ethenogenes* isolated and characterized by Maymó-Gatell et al. (*Science,* 176:1568 (1997)).

The terms "DHE-PL, DHE-STF, DHE-DAB, DHE-DLL and DHE-PIN" will refer to strains of *Dehalococcoides sp.* containing the instant dechlorinating 16S rDNA profile.

The term "dechlorinating bacteria" refers to any bacterial species or strain that has the ability to remove at least one chlorine atom from a chlorinated organic compound. Dechlorinating bacteria may have the ability to grow on chlorinated organics as a sole carbon source, or may prefer degradation using an alternate energy source.

The term "chlorinated compounds" will mean any straight chain or ring containing organic compound which contains at least one chlorine atom.

Trichloroethylene will be abbreviated "TCE".

Perchloroethylene will be abbreviated "PCE".

The term "16S rDNA" will refer to the DNA encoding ribosomal RNA found within bacterial cells.

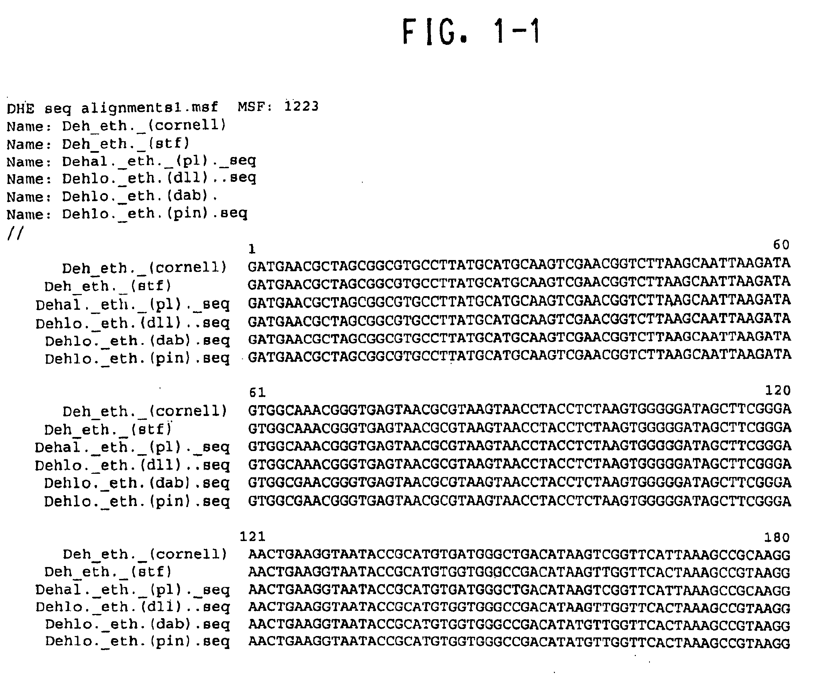

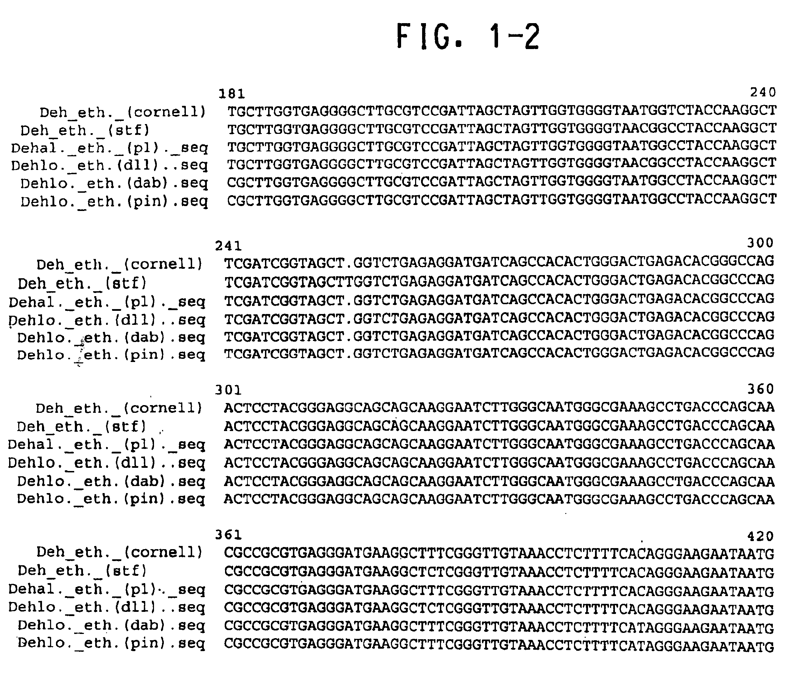

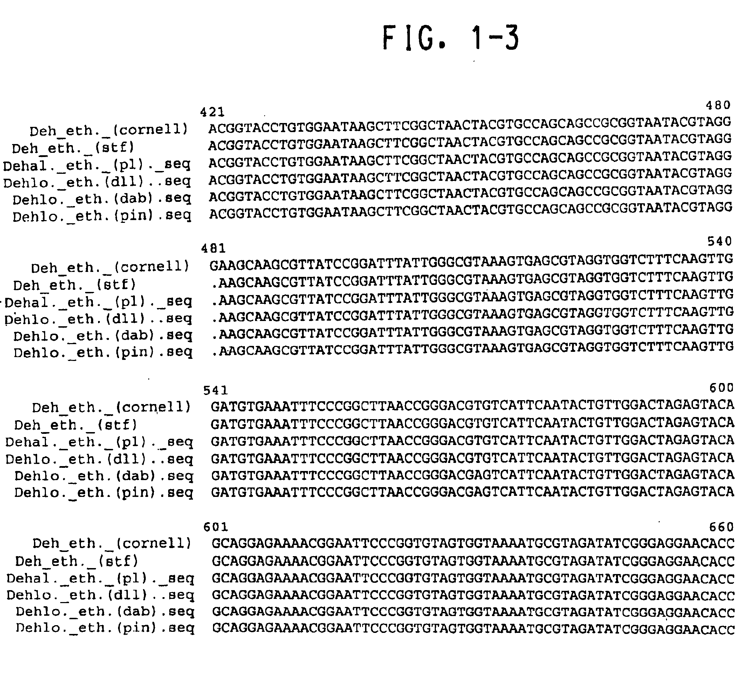

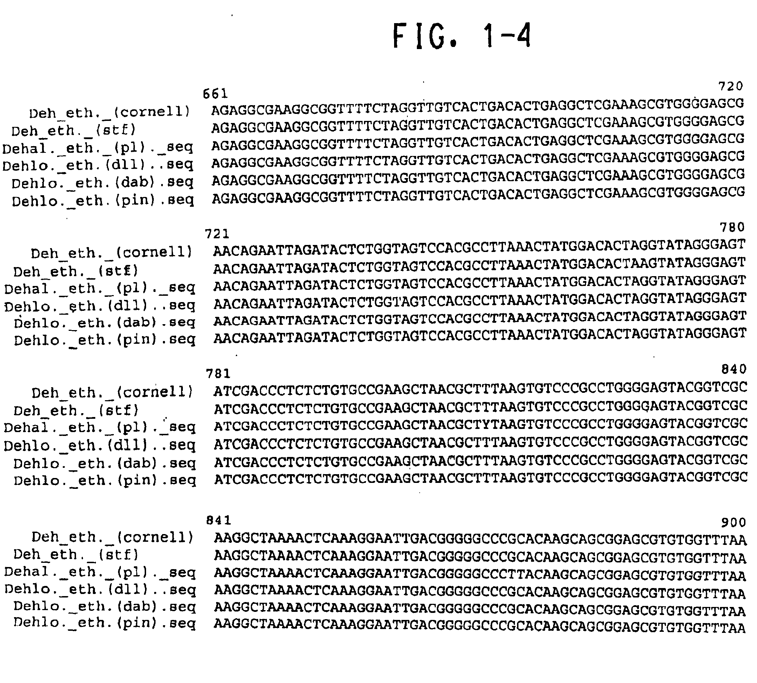

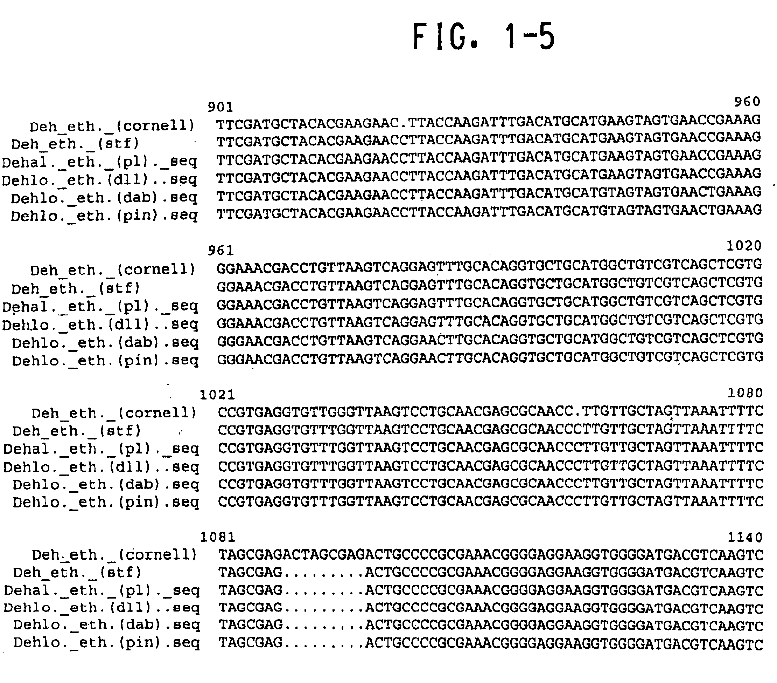

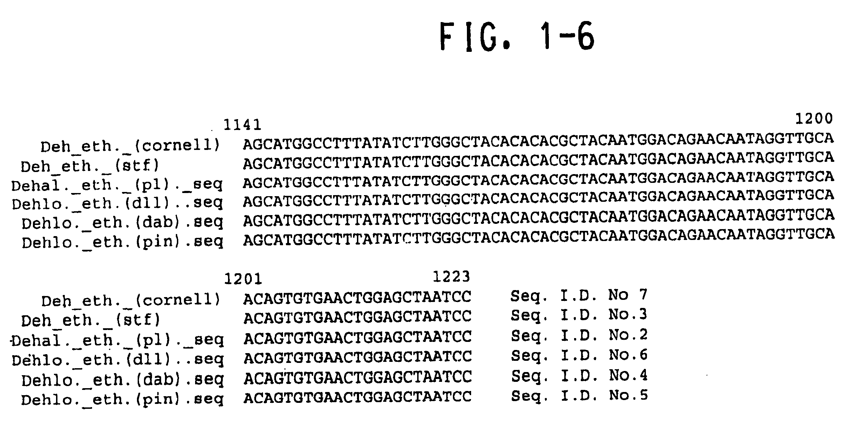

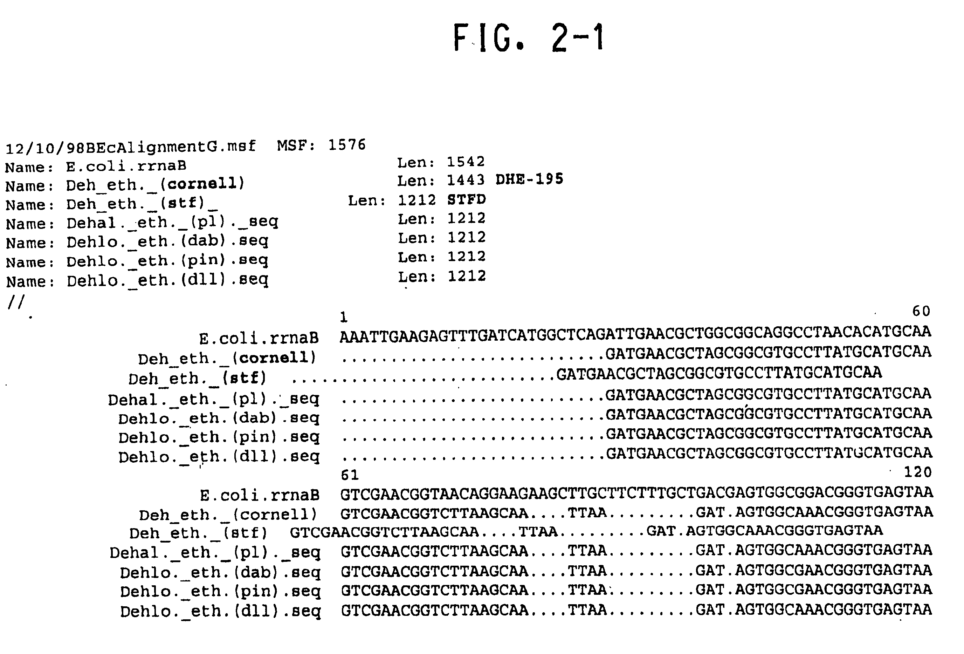

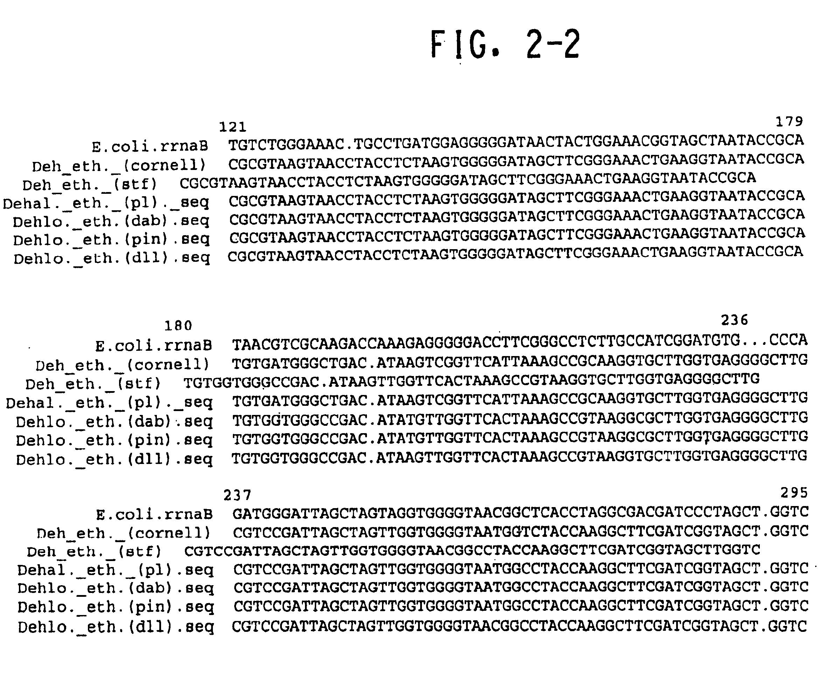

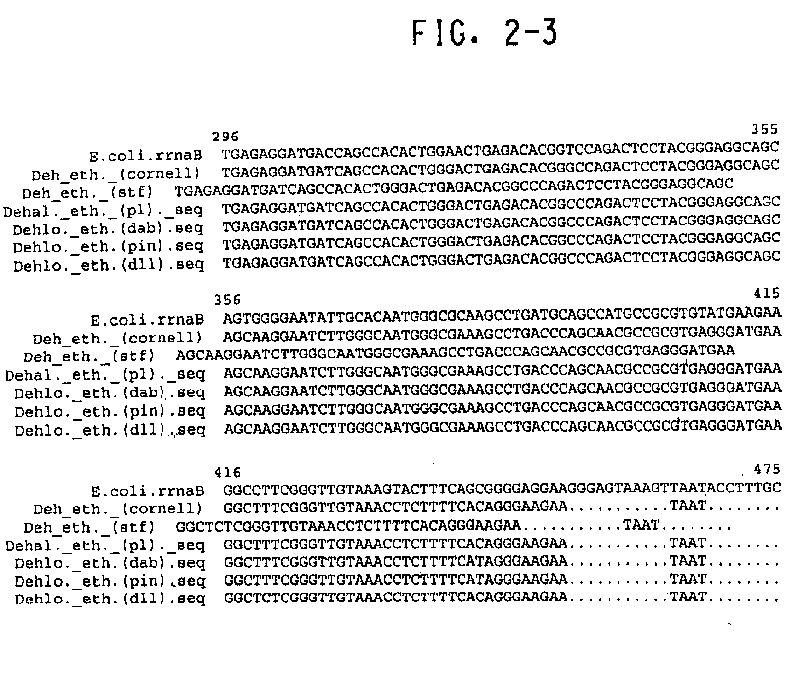

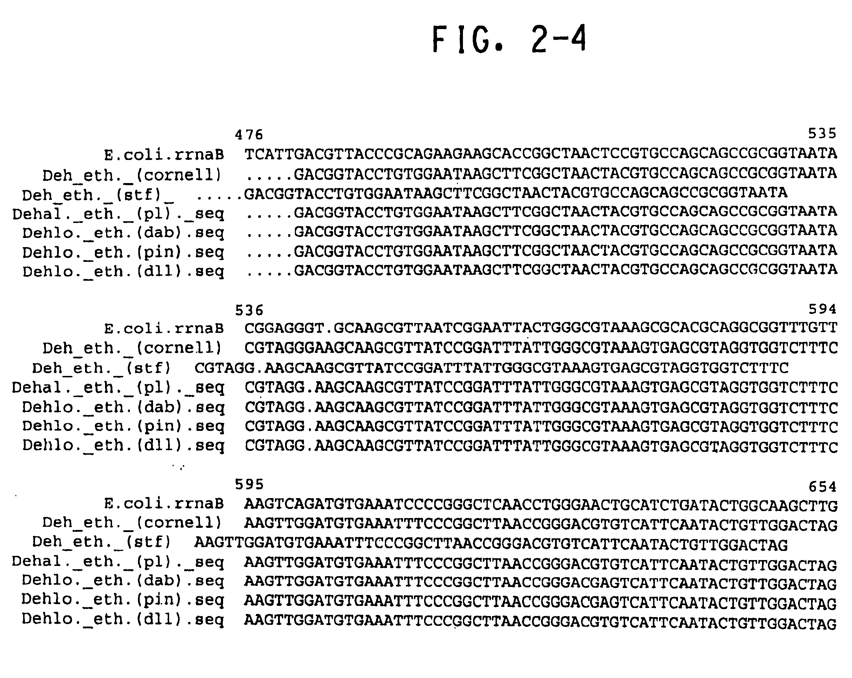

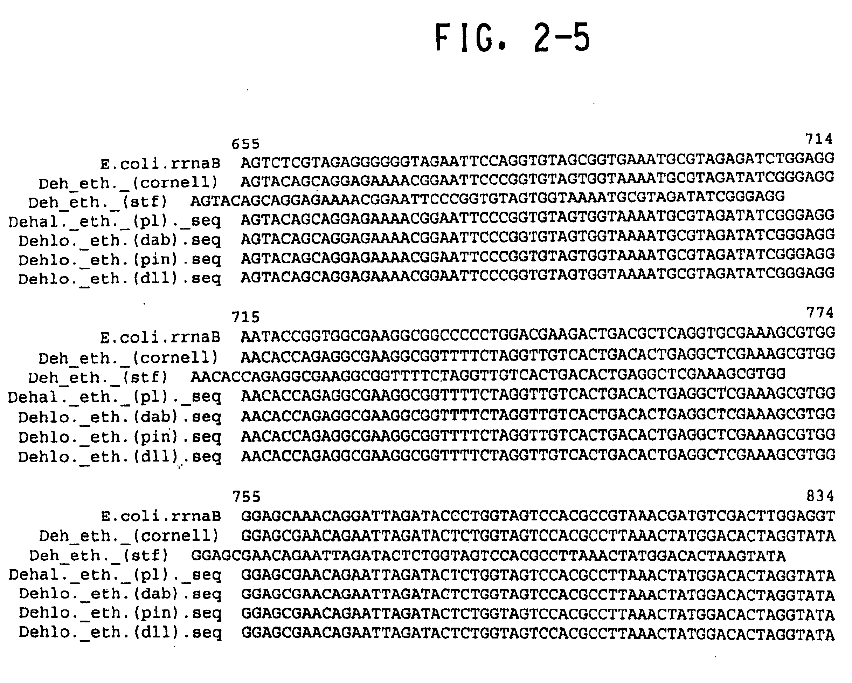

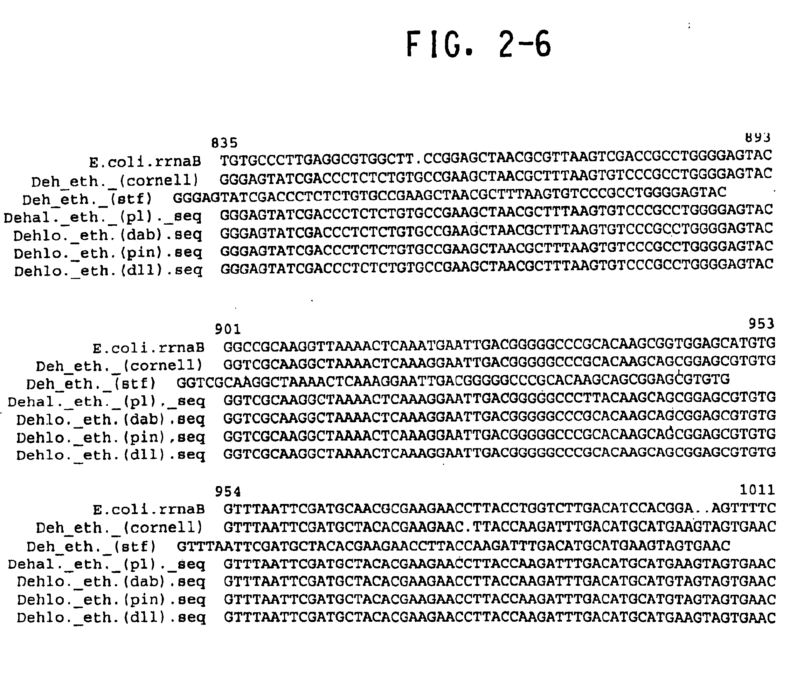

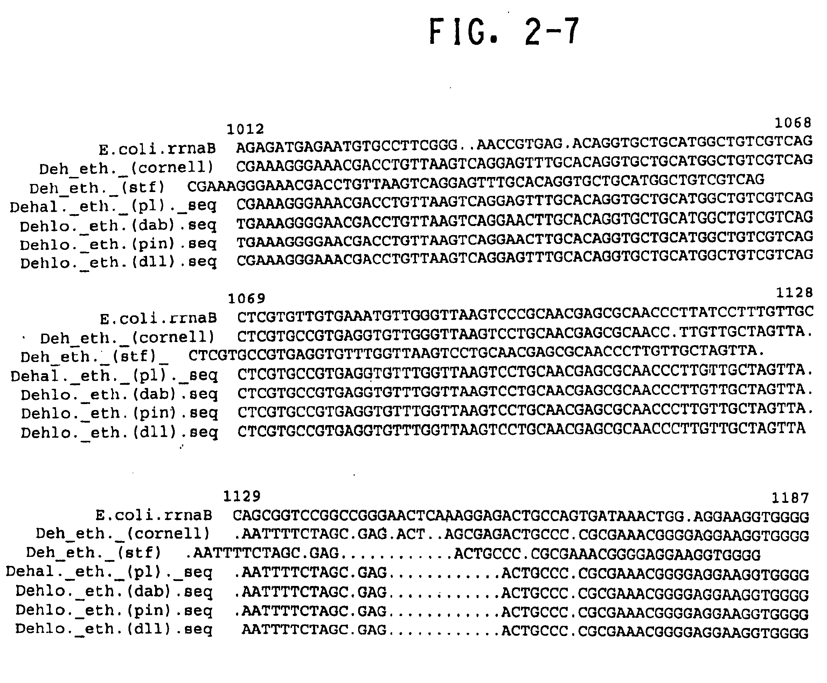

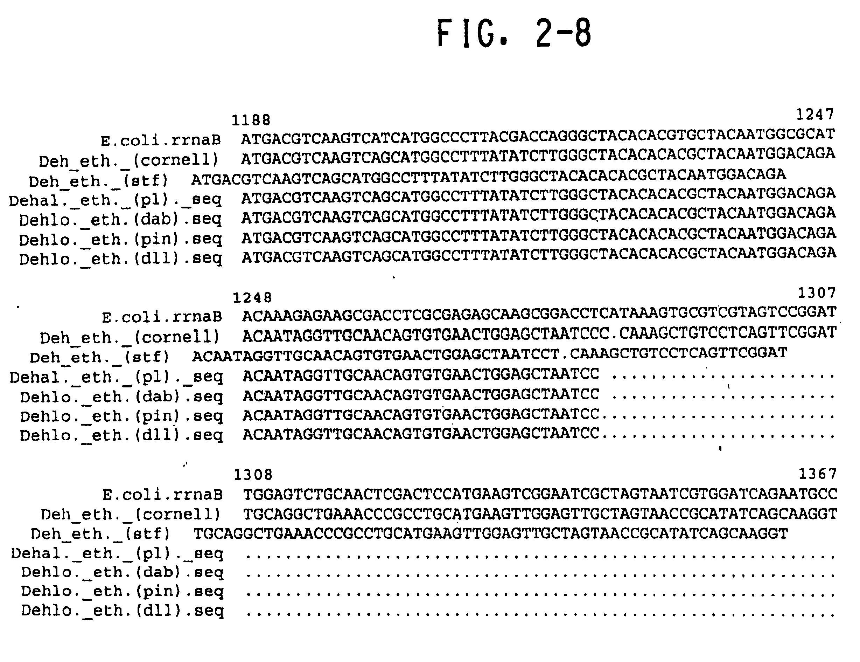

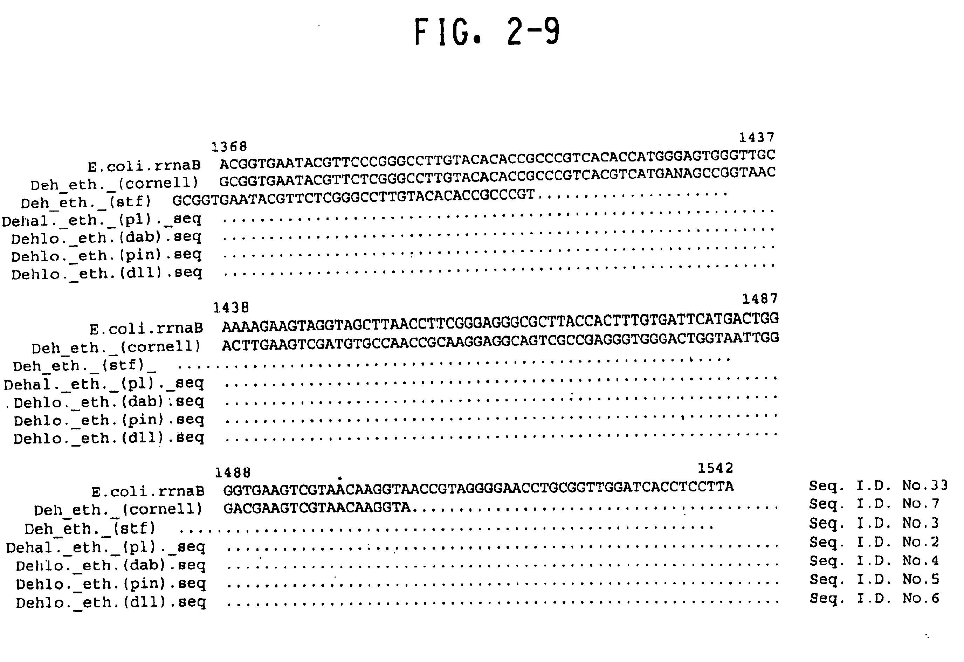

The term "16S rDNA profile" will refer to the specific DNA sequence of the rDNA gene in any particular organism. For the purposes of the present invention the 16S rDNA profiles for DHE-195, DHE-PL, DHE-STF, DHE-DAB, DHE-DLL and DHE-PIN are illustrated in Figures 1 and 2.

The term "signature sequence" or signature sequence region" will refer to those short sequences in the 16S gene or rRNA molecule which are unique to a certain group or groups of organisms. These sequences can be used to define domains, group, subdivisions genera or species of an organism.

The term "consensus sequence" as used herein, as it relates to the alignment of a given set of sequences, will be defined as the sequence of the set of bases where a designated base is the one that occurs most often at each position in the 16S sequence.

The term "reference sequence" as used herein, as it relates to the alignment of a given set of sequences, will be defined as the particular 16S sequence to which the bases at each position of an alignment of 16S sequences are compared. The reference sequence used herein was an *E. coli* 16S rDNA sequence. Bases identified in the reference sequence that correlate to corresponding bases in a 16S rDNA profiled are assigned an "E number". Thus, the base labeled E-28 on the reference sequence corresponds to base 1 of the 16S rDNA profile of DHE-195 and E-107 corresponds to base 66 of DHE-195. The complete correlation is given in Table 2.

The term "dechlorinating 16S rDNA profile" will refer to a 16S rDNA profile containing the diagnostic sequence as set forth in SEQ ID NO:1.

The term "diagnostic sequence" will refer to the sequence ATTTTCTAGCGAGACTGCCCCGCG (SEQ ID NO:1) which is indicative of dechlorinating activity.

The letters "A", "G", "T", "C" when referred to in the context of nucleic acids will mean the purine bases Adenine (C5H5N5), Guanine (C5H5N5O) and the pyrimidine bases Thymine (C5H6N2O2) and Cytosine (C4H5N3O) respectively.

In this disclosure, a number of terms and abbreviations are used. The following definitions are provided.

"Gene" refers to a nucleic acid fragment that expresses a specific protein, including regulatory sequences preceding (5' non-coding sequences) and following (3' non-coding sequences) the coding sequence.

The term "nucleic acid fragment" will refer to a polymer of RNA or DNA that is single- or double-stranded, optionally containing synthetic, non-natural or altered nucleotide bases. A nucleic acid fragment in the form of a polymer of DNA may be comprised of one or more segments of cDNA, genomic DNA or synthetic DNA.

The term "oligonucleotide" refers to primers, probes, oligomer fragments to be detected, labeled-replication blocking probes, oligomer controls, and shall be generic to polydeflxyribonucleotides (containing 2-deoxy-D-ribose), to polyribonucleotides (containing D-nbose) and to any polynucleotide which is an N glycoside of a purine or pyrimidine base (nucleotide), or modified purine or pyrimidine base. Also included in the definition of "oligonucleotide" are nucleic acid analogs (e.g., peptide nucleic acids) and those that have been structurally modified (e.g., phosphorothioate linkages). There is no intended distinction between the length of a "nucleic acid", "polynucleotide" or an "oligonucleotide".

The term "primer" refers to an oligonucleotide (synthetic or occurring naturally), which is capable of acting as a point of initiation of nucleic acid synthesis or replication along a complementary strand when placed under conditions in which synthesis of a complementary stand is catalyzed by a polymerase.

The term "probe" refers to an oligonucleotide (synthetic or occurring naturally), that is significantly complementary to a "fragment" and forms a duplexed structure by hybridization with at least one strand of the fragment.

The term "complementary" is used to describe the relationship between nucleotide bases that are hybridizable to one another. For example, with respect to DNA, adenosine is complementary to thymine and cytosine is complementary to guanine.

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength. Hybridization and washing conditions are well known and exemplified in Sambrook, J., Fritsch, E. F. and Maniatis, T. Molecular Cloning: A Laboratory Manual, Second Edition, Cold Spring Harbor Laboratory Press, Cold Spring Harbor (1989), particularly Chapter 11 and Table 11.1 therein. The conditions of temperature and ionic strength determine the "stringency" of the hybridization. For preliminary screening for homologous nucleic acids, low stringency hybridization conditions, corresponding to a Tm of 55°, can be used, e.g., 5X SSC, 0.1% SDS, 0.25% milk, and no formamide; or 30% formamide, 5X SSC, 0.5% SDS. Moderate stringency hybridization conditions correspond to a higher Tm, e.g., 40% formamide, with 5X or 6X SSC.

Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et al., *supra,* 9.50-9.51). For hybridizations with shorter nucleic acids, i.e., oligonucleotides, the position of mismatches becomes more important, and the length of the oligonucleotide determines its specificity (see Sambrook et al., *supra,* 11.7-11.8). In one embodiment the length for a hybridizable nucleic acid is at least about 10 nucleotides. Preferable a minimum length for a hybridizable nucleic acid is at least about 15 contiguous nucleotides; more preferably at least about 20 contiguous nucleotides; and most preferably the length is at least 30 contiguous nucleotides. Thus, where a "probe" or "primer" is "derived from" or corresponds to a "portion" of a nucleic acid fragment, the probe or primer or portion will preferably be at least about 15 contiguous nucleotides; more preferably at least about 20 contiguous nucleotides; and most preferably the length is at least 30 contiguous nucleotides of the fragment from which it is derived. Furthermore, the skilled artisan will recognize that the temperature and wash solution salt concentration may be adjusted as necessary according to factors such as length of the probe.

The term "amplification product" refers to portions of nucleic acid fragments that are produced during a primer directed amplification reaction. Typical methods of primer directed amplification include polymerase chain reaction (PCR), ligase chain reaction (LCR) or Strand displacement Amplification (SDA). If PCR methodology is selected, the replication composition would include for example, nucleotide triphosphates, two primers with appropriate sequences, DNA or RNA polymerase and proteins. These reagents and details describing procedures for their use in amplifying nucleic acids are provided in U.S. Patent No. 4,683,202 (1987, Mullis, et al.) and U.S. Patent No. 4,683,195 (1986, Mullis, et al.). If LCR methodology is selected, then the nucleic acid replication compositions would comprise, for example, a thernnostable ligase, e.g., *T. aquaticus* ligase, two sets of adjacent oligonucleotides wherein one member of each set is complementary to each of the target strands, Tris HCl buffer, KCl, EDTA, NAD, dithiothreitol and salmon sperm DNA. See, for example, Tabor et al., *Proc. Acad Sci. U.S.A.,* 82, 1074-1078 (1985)).

The term "sequence analysis software" refers to any computer algorithm or software program that is useful for the analysis of nucleotide or amino acid sequences. "Sequence analysis software" may be commercially available or independently developed. Typical sequence analysis software will include but is not limited to the GCG suite of programs (Wisconsin Package Version 9.0, Genetics Computer Group (GCG), Madison, Wisc.), BLASTP, BLASTN, BLASTX (Altschul et al., *J*. *Mol. Biol.* 215:403-410 (1990), and DNASTAR (DNASTAR, Inc., 1228 S. Park St. Madison, WI 53715 USA). Within the context of this application it will be understood that where sequence analysis software is used for analysis, that the results of the analysis will be based on the "default values" of the program referenced, unless otherwise specified. As used herein "default vales" will mean any set of values or parameters which originally load with the software when first initialized.

Standard recombinant DNA and molecular cloning techniques used here are well known in the art and are described by Sambrook, J., Fritsch, E. F. and Maniatis, T., Molecular Cloning: A Laboratory Manual, Second Edition, Cold Spring Harbor Laboratory Press, Cold Spring Harbor, NY (1989) (hereinafter "Maniatis"); and by Silhavy, T. J., Bennan, M. L. and Enquist, L. W., Experiments with Gene Fusions, Cold Spring Harbor Laboratory Cold Press Spring Harbor, NY (1984); and by Ausubel, F. M. et al., Current Protocols in Molecular Biology, published by Greene Publishing Assoc. and Wiley-Interscience (1987).

The present invention relates to unique 16S rDNA sequences which have been isolated from the bacteria very similar if not related to *Dehalococcoides ethenogenes,* which are associated with the ability of this bacteria to dechlorinate chlorinated organic compounds. The sequences were isolated from bacteria found in soil samples of various industrial sites that have been shown to contain bacteria that have the ability to dechlorinate chlorinated compounds. The sequences are useful for the identification new dechlorinating bacteria, as well as for sub-typing strains of *Dehalococcoides ethenogenes.*

Dechlorinating bacteria were isolated from the aquifer soil taken from around industrial sites by means well known in the art. Samples were maintained under anaerobic conditions and cultured in a suitable medium for the growth of anaerobic soil bacteria. Such culture procedures and media are common and well known in the art and are described in Manual of Methods for General Bacteriology (Phillipp Gerhardt, R. G. E. Murray, Ralph N. Costilow, Eugene W. Nester, Willis A. Wood, Noel R. Krieg and G. Briggs Phillips, eds), American Society for Microbiology, Washington, DC. (1994)) or by Thomas D. Brock in Biotechnology: A Textbook of Industrial Microbiology, Second Edition, Sinauer Associates, Inc., Sunderland, MA (1989).

In order to enrich the cultured soil samples for dechlorinating bacteria, the samples were contacted with a low level of chlorinated organic compound. A number of chlorinated compounds are suitable for this purpose, including, but not limited to carbontetrachloride, tetrachloroethene, chloroform, dichloromethane, trichloroethene, dichloroethylene, vinyl chloride, and chloroaromatics, where chlorinated ethenes are preferred and TCE and PCE are preferred. Incubation proceeded for about six months, and cultures were analyzed periodically for the disappearance of the chlorinated organic and the appearance of degradation products. Cultures demonstrating the ability to degrade chlorinated organics, were selected for further analysis.

Bacteria from dechlorinating cultures were removed by standard methods and total chromosomal DNA was isolated from the microorganisms through a bead mill homogenization procedure. A fragment of the 16S rRNA gene was amplified from the genomic DNA extract by PCR using 16S rDNA primers specific for dechlorinating microbes. The 16S rDNA PCR product was cloned and sequenced to confirm its identity (M. I. More et al. 1994. *Appl. Environ. Microbiol.,* 60, 1572-1580). Each raw 16S sequences obtained were assembled into a contig, and a consensus was manually constructed using Seqman II in DNAstar (DNAstar, Inc., Madison, WI). For each test sequence, a Pearson and Lipman similarity search was performed using the FASTA program in GCG (Wisconsin Package Version 9.0, Genetics Computer Group, Madison, WI). The nearest organism in similarity in 16S rRNA sequence to the test sequence was used as the nearest match for identification. Those 16S DNA gene sequences that were identified to be similar to the dechlorinating bacteria, *Dehalococcoides ethenogenes* DHE-195 (GenBank Accession No. AF004928), were aligned with selected 16s rRNA sequences extracted from the Ribosomal Database Project (Michigan State University) that were a representation of the major microorganism domains, Bacteria and Archeae in the Universal Phylogenetic Tree of Life. The sequences were aligned using MegAlign in DNAstar, using the default software parameters. From this alignment probable region for signature sequences were mapped. Then sequences from each region were tested against the Ribosomal Database (RDB) for unique sequences that could be signature sequences and utilized as PCR primes or detection probes.

Within the 16S rDNA profile defined by the comparison of the isolated dechlorinators, (see Figures 1 and 2) three signature regions showed considerable variation from the known sequences. Those regions were defined as extending from E1146 to E1156 (SEQ ID NO:1),from E180 to E227 (SEQ ID NO:8), and form E1001 to E1047 (SEQ ID NO: 30). All of the dechlorinating isolates of the present invention contained the sequence as set forth in SEQ ID NO:1, which is conspicuously absent from the sequence known in the art (Maymó-Gatell et al. (*Science,* 176:1568 (1997)).

Although a region similar to that defined by SEQ ID NO:8 is found in the literature sequence, there are significant variations at positions, E184, E190, E197, E200, E207, E216, and E221 as shown below in Formula I.

With in the context the present invention Applicants have discovered that within the signature region defined by SEQ ID NO:8 and Formula I above, the R at position E184 may be A/G, the Y at position E190 may be C/T, the W at position E198 may be A/T, and the Y's at position E201, E208, E217, and E222 may be T/C.

Similarly the region defined by SEQ ID NO:30 is also found in the literature but contains significant variations at positions, E1003, E1012, E1020, E1039, and E1040 as shown below in Formula II.

As with SEQ ID NO:8, Applicants have discovered that within the signature region defined by SEQ ID NO:30 and Formula II above, the W at position E1003 may be A/T, at position E1012 the M may be A/C, at position E1020 the R may be A/G, at position E1039 the R may be A/G and , at position E1040 the M may be A/C.

Likewise, if the entire 16S rDNA profile is examined, it is seen that there are significant single base differences throughout the entire profile (Figures 1 and 2). These differences are illustrated in tabular form in Table 2. Accordingly a 16S rDNA profile sequence, having the following bases substitutions taken independently or together will be diagnostic for dechlorinating bacteria: E107=G, base E184=G, base E190=C, E 198=T, E201= T, E208=C, E217=T, E222=C, E264=C, E267=C, E291=T, E333= C, E420=C, E444=T, E631=A, E829=A, E933=T, E934=T, E980=C, E 1003=T, E1012=T, E1020=G, E1039=A, E1040=C, E1087=T, and E1114=C.

### Assay Methods

The instant sequences may be used in a variety of formats for the detection of dechlorinating bacteria. The two most convenient formats will rely on methods of nucleic acid hybridization or primer directed amplification methods such as PCR.

### Nucleic Acid Hybridization Methods

The basic components of a nucleic acid hybridization test include a probe, a sample suspected of containing a dechlorinating bacteria and a specific hybridization method. As noted above, probes of the present invention are single strand nucleic acid sequence which is complementary to the nucleic acid sequences to be detected. Probes are "hybridizable" to the nucleic acid sequence to be detected. The probe length can vary from 5 bases to tens of thousands of bases, and will depend upon the specific test to be done. Only part of the probe molecule need be complementary to the nucleic acid sequence to be detected. In addition, the complementarity between the probe and the target sequence need not be perfect. Hybridization does occur between imperfectly complementary molecules with the result that a certain fraction of the bases in the hybridized region are not paired with the proper complementary base. A probe may be composed of either RNA or DNA. The form of the nucleic acid probe may be a marked single strand molecule of just one polarity or marked single strand molecule having both polarities present. The form of the probe, like its length, will be determined by the type of hybridization test to be done.

The sample may or may not contain the organism of interest. The sample may take a variety of forms, including liquid such as water, or solid such as dust, or soil. The sample nucleic acid must be made available to contact the probe before any hybridization of probe and target molecule can occur. Thus the organism's RNA must be free from the cell and placed under the proper conditions before hybridization can occur. Methods of in solution hybridization necessitate the purification of the RNA in order to be able to obtain hybridization of the sample rRNA with the probe. This has meant that to utilize the in solution method for detecting target sequences in a sample, the nucleic acids of the sample must first be purified to eliminate protein, lipids, and other cell components, and then contacted with the probe under hybridization conditions. Method for the purification of the sample nucleic acid are common and well known in the art (Maniatis, *supra*).

Similarly, hybridization methods are well defined. Typically the probe and sample must be mixed under conditions which will permit nucleic acid hybridization. This involves contacting the probe and sample in the presence of an inorganic or organic salt under the proper concentration and temperature conditions. The probe and sample nucleic acids must be in contact for a long enough time that any possible hybridization between the probe and sample nucleic acid may occur. The concentration of probe or target in the mixture will determine the time necessary for hybridization to occur. The higher the probe or target concentration the shorter the hybridization incubation time needed.

In one embodiment, hybridization assays may be conducted directly on bacterial lysates, without the need to extract the nucleic acids. This eliminates several steps from the sample-handling process and speeds up the assay. To perform such assays on crude cell lysates, a chaotropic agent is typically added to the cell lysates prepared as described above. The chaotropic agent stabilizes nucleic acids by inhibiting nuclease activity. Furthermore, the chaotropic agent allows sensitive and stringent hybridization of short oligonucleotide probes to RNA at room temperature [Van Ness and Chen (1991) *Nucl. Acids Res.* 19:5143-5151]. Suitable chaotropic agents include guanidinium chloride, guanidinium thiocyanate, sodium thiocyanate, lithium tetrachloroacetate, sodium perchlorate, rubidium tetrachloroacetate, potassium iodide, and cesium trifluoroacetate, among others: Typically, the chaotropic agent will be present at a final concentration of about 3M. If desired, one can add formamide to the hybridization mixture, typically 30-50% (v/v).

Alternatively, one can purify the rRNA prior to probe hybridization. A variety of methods are known to one of skill in the art (e.g., phenol-chloroform extraction, IsoQuick extraction (MicroProbe Corp., Bothell, WA), and others). Pre-hybridization purification is particularly useful for standard filter hybridization assays. Furthermore, purification facilitates measures to increase the assay sensitivity by incorporating *in vitro* RNA amplification methods such as self-sustained sequence replication (see for example Fahy et al. (1991) in PCR Methods and Applications. Cold Spring Harbor Laboratory Press, pp. 25-33) or reverse transcriptase PCR (Kawasaki (1990) in PCR Protocols: A Guide to Methods and Applications, M. A. Innis et al., eds., pp. 21-27). One can obtain amplified rRNA by using in vitro RNA amplification techniques as described in Fahy et al., *supra.;* Kawasaki, *supra.* The exact procedure used is not crucial, provided that it does not amplify significant amounts of DNA, which would tend to obscure results.

Once the pre-rRNA is released from the cells, it can be detected by any of a variety of methods. The method of rRNA detection is not crucial to the invention. However, the most useful embodiments have at least some of characteristics of speed, convenience, sensitivity, and specificity. Direct DNA probe analysis is suitable, as is an *in vitro* RNA amplification method, such as 3SR, that employs labelled primers.

Various hybridization solutions can be employed. Typically, these comprise from about 20 to 60% volume, preferably 30%, of a polar organic solvent. A common hybridization solution employs about 30-50% v/v formamide, about 0.15 to 1M sodium chloride, about 0.05 to 0.1 M buffers, such as sodium citrate, Tris-HCl, PIPES or HEPES (pH range about 6-9), about 0.05 to 0.2% detergent, such as sodium dodecylsulfate, or between 0.5-20 mM EDTA, FICOLL (Pharmacia Inc.) (about 300-500 kilodaltons), polyvinylpyrrolidone (about 250-500 kdal), and serum albumin. Also included in the typical hybridization solution will be unlabeled carrier nucleic acids from about 0.1 to 5 mg/mL, fragmented nucleic DNA, e.g., calf thymus or salmon sperm DNA, or yeast RNA, and optionally from about 0.5 to 2% wt./vol. glycine. Other additives may also be included, such as volume exclusion agents which include a variety of polar water-soluble or swellable agents, such as polyethylene glycol, anionic polymers such as polyacrylate or polymethylacrylate, and anionic saccharidic polymers, such as dextran sulfate.

Nucleic acid hybridization is adaptable to a variety of assay formats. One of the most suitable is the sandwich assay format. The sandwich assay is particularly adaptable to hybridization under non-denaturing conditions. A primary component of a sandwich-type assay is a solid support. The solid support has adsorbed to it or covalently coupled to it immobilized nucleic acid probe that is unlabeled and complementary to one portion of the rRNA sequence. Preferred are those probes that hybridize to regions of the rRNA that have minimal secondary and tertiary interactions. The advantage of such probes is that the hybridization can be carried out without the additional step of heat denaturing the sample nucleic acid. For example, the hybridization can be carried out at room temperature.

The sandwich assay may be encompassed in an assay kit. This kit would include a first component for the collection of samples from soil such as vials for containment, and buffers for the disbursement and lysis of the sample. A second component would include media in either dry or liquid form for the hybridization of target and probe polynucleotides, as well as for the removal of undesirable and nonduplexed forms by washing. A third component includes a solid support (dipstick) upon which is fixed or to which is conjugated unlabeled nucleic acid probe(s) that is(are) complementary to a part of the precursor rRNA of the species of bacteria being tested. In the case of multiple target analysis more than one capture probe, each specific for its own rRNA, will be applied to different discrete regions of the dipstick. A fourth component would contain labeled probe that is complementary to a second and different region of the same rRNA strand to which the immobilized, unlabeled nucleic acid probe of the third component is hybridized.

In another embodiment, the instant 16S rDNA sequence may be used as a 3' blocked detection probe in either a homogeneous or heterogeneous assay format. For example a probe generated from the instant sequences may be 3' blocked or non-participatory and will not be extended by, or participate in, a nucleic acid amplification reaction. Additionally, the probe incorporates a label that can serve as a reactive ligand that acts as a point of attachment for the immobilization of the probe/analyte hybrid or as a reporter to produce detectable signal. Accordingly, genomic or cDNA isolated from the test organism is amplified by standard primer-directed amplification protocols in the presence of an excess of the 16S rDNA 3' blocked detection probe to produce amplification products. Because the probe is 3' blocked, it does not participate or interfere with the amplification of the target. After the final amplification cycle, the detection probe anneals to the relevant portion of the amplified DNA and the annealed complex is then captured on a support through the reactive ligand.

### PCR Assay Methods

In an alternate embodiment the present sequences may be used as primers or to generate primers that may be used in primer directed nucleic acid amplification to detect the presence of dechlorinating bacteria. A variety of primer directed nucleic acid amplification methods are known in the art including thermal cycling methods such as polymerase chain reaction (PCR) and ligase chain reaction (LCR) as well as isothermal methods and strand displacement amplification (SDA). The preferred method is PCR. Typically, in PCR-type amplification techniques, the primers have different sequences and are not complementary to each other. Depending on the desired test conditions, the sequences of the primers should be designed to provide for both efficient and faithful replication of the target nucleic acid. Methods of PCR primer design are common and well known in the art. (Thein and Wallace, "The use of oligonucleotide as specific hybridization probes in the Diagnosis of Genetic Disorders", in *Human Genetic Diseases: A Practical Approach,* K. E. Davis Ed, (1986) pp. 33-50 IRL Press, Herndon, Virginia); Rychlik, W. (1993) In White, B. A. (ed.), Methods in Molecular Biology, Vol. 15, pages 31-39, PCR Protocols: Current Methods and Applications. Humania Press, Inc., Totowa, NJ.)

If a nucleic acid target is to be exponentially amplified, then two primers are used each having regions complementary to only one of the stands in the target. After heat denaturation, the single-stranded target fragments bind to the respective primers which are present in excess. Both primers contain asymmetric restriction enzyme recognition sequences located 5' to the target binding sequences. Each primer-target complex cycles through nicking and polymerization/displacement steps in the presence of a restriction enzyme, a DNA polymerase and the three dNTP's and one dNTP[aS] as discussed above. An in depth discussion of SDA methodology is given by Walker et al., *Proc. Natl. Acad. Sci. U.S.A.,* 89, 392, (1992).

Alternatively, asymmetric amplification can be used to generate the strand complementary to the detection probe. Asymmetric PCR conditions for producing single-stranded DNA would include similar conditions for PCR as described however, the primer concentrations are changed with 50 pmol of the excess primer and 1 pmol of the limiting primer. It is contemplated that this procedure would increase the sensitivity of the method. This improvement in sensitivity would occur by increasing the number of available single strands for binding with the detection probe.

Within the context of the present invention primers will be designed to conserved regions of the 16S rDNA profile which are associated with dechlorination. The most significant of those regions are the sequences set forth in SEQ ID NO:8 and SEQ ID NO:30.

Following amplification and prior to sequencing, the amplified nucleotide sequence may be ligated to a suitable vector followed by transformation of a suitable host organism with said vector. One thereby ensures a more readily available supply of the amplified sequence. Alternatively, following amplification, the amplified sequence or a portion thereof may be chemically synthesized for use as a nucleotide probe. In either situation the DNA sequence of the variable region is established using methods such as the dideoxy method (Sanger, F. et al. *Proc. Natl. Acad. Sci* (1977) 74, 5463-5467). The sequence obtained is used to guide the choice of the probe for the organism and the most appropriate sequence(s) is/are selected.

### EXAMPLES

The present invention is further defined in the following Examples. It should be understood that these Examples, while indicating preferred embodiments of the invention, are given by way of illustration only. From the above discussion and these Examples, one skilled in the art can ascertain the essential characteristics of this invention, and can make various changes and modifications of the invention to adapt it to various usages and conditions.

### GENERAL METHODS

Standard recombinant DNA and molecular techniques used in the Examples are well known in the art. Techniques suitable for use in the following examples may be found in Sambrook, J., Fritsch, E. F. and Maniatis, T., Molecular Cloning: A Laboratory Manual, Second Edition, Cold Spring Harbor Laboratory Press, Cold Spring Harbor, NY (1989) (hereinafter "Maniatis").

Materials and methods suitable for the maintenance and growth of bacterial cultures are well known in the art. Techniques suitable for use in the following examples may be found as set out in Manual of Methods for General Bacteriology (Phillipp Gerhardt, R. G. E. Murray, Ralph N. Costilow, Eugene W. Nester, Willis A. Wood, Noel R. Krieg and G. Briggs Phillips, eds), American Society for Microbiology, Washington, DC. (1994)) or by Thomas D. Brock in Biotechnology: A Textbook of Industrial Microbiology, Second Edition, Sinauer Associates, Inc., Sunderland, MA (1989). All reagents, restriction enzymes and materials used for the growth and maintenance of bacterial cells were obtained from Aldrich Chemicals (Milwaukee, WI), DIFCO Laboratories (Detroit, MI), GIBCO/BRL (Gaithersburg, MD), or Sigma Chemical Company (St. Louis, MO) unless otherwise specified.

Manipulations of genetic sequences were accomplished using the suite of programs available from the Genetics Computer Group Inc. (Wisconsin Package Version 9.0, Genetics Computer Group (GCG), Madison, WI), DNASTAR (DNASTAR, Inc. 1228 S. Park St. Madison, W153715 USA), or the "on-line" Probe Match Program from the Ribosomal Database Project II (Michigan State University, East Lansing, MI). Where any sequence analysis software was used in the following examples, default values were used unless otherwise specified.

The meaning of abbreviations is as follows: "h" means hour(s), "min" means minute(s), "sec" means second(s), "d" means day(s), "mL" means milliliters, "L" means liters.

### EXAMPLE 1

### Isolation And Characterization Of Dechlorinating Soil Organisms

Aquifer core samples were obtained by split spoon sampling at depths ranging from 10 to 80 ft, depending on the depth of the particular aquifer to be tested. The cores were taken in sterile stainless steel cylinders or placed in sterile glass vials. The core samples were immediately shipped to the laboratory at ambient temperatures and under anaerobic conditions. Upon arrival the samples were stored in an anaerobic glove bag (chamber) (Coy Laboratory Products Inc., Ann Arbor, MI), whose atmosphere was 10% H₂, 5% CO₂ and 85% N₂.

The laboratory microcosms were prepared in 250 mL Wheaton bottles (Wheaton Co., Millville, NJ) within the anaerobic chamber. Duplicate microcosms were prepared for the following conditions: Killed Control (live soil autoclaved for 1 hr on 2 consecutive days), Live soil, and Live soil + 0.05% yeast extract. Each microcosm contains 20% soil and 80% BTZ-3 media (NH₄Cl, 4.3 g/L; KH₂PO₄, 50 g/L; MgCl-6H₂O, 20 g/L; CaCl₂-2H₂O, 1 g/L; HEPES, 50 mM/L; mineral solution, 10 mL/L; resazurin 0.2%, 5 mL/L). The microcosm were filled to top such there was little or no headspace, and then stoppered with Teflon™ lined disks and crimp-sealed with aluminum seals (Wheaton Co., Millville, NJ). The resazurin addition permitted the visualization of low potential anaerobic conditions by a color change from pink to colorless. Each microcosm was spiked with 5 ppm from a PCE or TCE solution saturated in water. The microcosms were incubated on their sides in the anaerobic chamber, in the dark, at ambient room temperature (22°C) for up to 180 days.

Samples were analyzed the next day as time zero (to) and then twice a week for the dechlorination of PCE or TCE and the formation of cisDCE, vinyl chloride or methane. All samples were taken in the anaerobic chamber by using a syringe mounted with a 23 gauge needle was use to puncture the Teflon™ septa to obtain a 5 mL liquid sample that was injected into a 10 mL headspace vial. Samples were tested using HP Headspace sampler 7694, HP5890 series II GC (FID detector, HP 5 capillary column #19091J-215), HP3365 Chemstation version A.03.34.

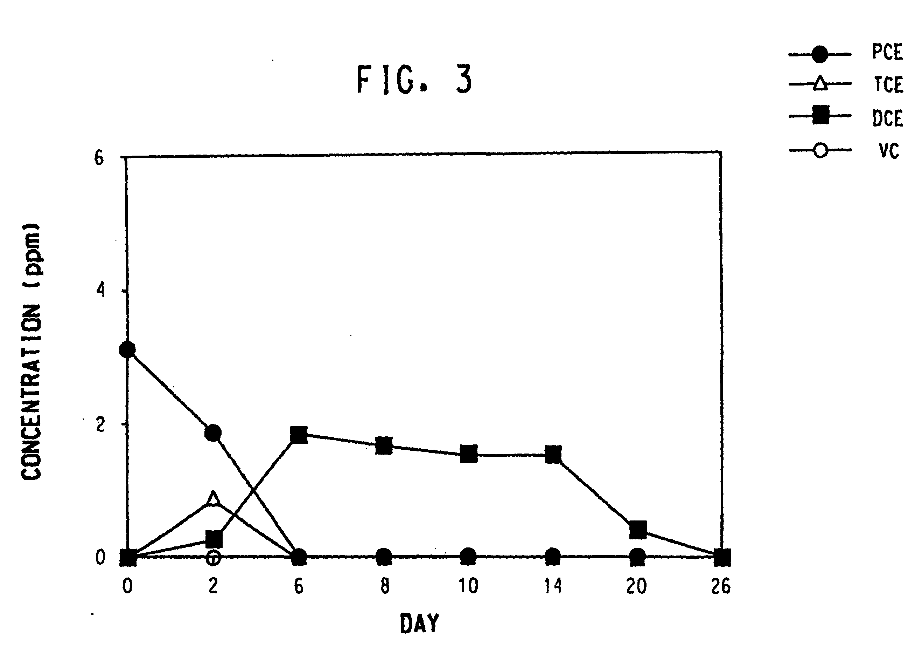

Figure 3 plots the concentration (parts per million; ppm) of chloroethenes in the microcosm medium as a function of time (days) and illustrates the dechlorination of chloroethenes. Dechlorination of PCE to TCE could be detected by GC/FID. Within two days with the formation of cisDCE from the dechlorination of TCE was detected. These results are found in the microcosms that has been amended with 0.05% yeast extract plus minimal salts media (BTZ-3 media). These results can also be seen in the microcosms that are amended with the minimal salts media alone. The difference is the dechlorination is slightly delayed. It takes four days before cisDCE is detected. Degradation of cisDCE would occur over the next two weeks. Vinyl chloride and ethene could only be detected at trace levels. The "Killed" control, did not show degradation of PCE or TCE during the duration of the experiment. Cell growth was shown by increase in the turbidity of the microcosm medium and by microscopic analysis.

### EXAMPLE 2

### Generation of PCR Primers and Probes for the Amplification and Detection of the Dehalococcoides Ethenogenes 16S rRNA Profiled

The detection and sequencing of the *Dehalococcoides ethenogenes*-like organisms used the set of PCR primers are shown in Table 1. The PCR primers were designed using signature sequence regions. To determine the location of these signature sequence, the *Dehalococcoides ethenogenes* sequence (GenBank No. AF004928)[SEQ ID NO:7] was aligned using MEGALIGN (DNAstar, Madison, WI or Pileup (Genetics Computer Group, Madison, WI) with 16S rRNA sequences from 100 organisms that represent most major domains, families and genera in the major kingdoms of Bacteria and Archaea. The conserved, variable, and highly variable regions could be delineated by boxing off the consensus sequences. Primer candidate sequences were manually picked from the variable and highly variable regions and then their uniqueness was determined by determining their potential as probes to a ribosomal sequence database sequences using the "on-line" Probe Match Program from the Ribosomal Database Project II (httpJ/www.cme.msu.edu/RDP/html/index.html)RDPII, Michigan State University, East Lansing, MI). This analysis returned an overview of the matches between a probe and its potential target sequence, as a listing and as a phylogenetic overview. The program results showed the sequences that match the query sequence (if there are such sequences) and also showed sequences that had mismatches, deletions and insertions, citing the number and positions of the aberrations.

The sequences which were unique and passed this test as signature sequences were then designed as either a forward or reverse primer, usually dependent on their position in the sequence. The most unique sequence of the signature sequence (specificity) was designed into the 3' end in either type of primer. The selected primers are shown in Table 1.

The primers were synthesized using standard β-cyanoethyl phosphoramidite coupling chemistry on controlled pore glass (CPG) supports on automated DNA oligonucleotide synthesizer (Applied Biosystems Model 392, Perkin-Elmer, Foster City, CA)

The primers were tested after they were synthesized using PCR on samples taken from microcosms known to have *Dehalococcoides ethenogenes*-like organisms. The PCR products were sized on agarose electrophoresis and then cloned and sequenced to verify that the amplified sequences were *Dehalococcoides ethenogenes*-like 16S rRNA sequences.

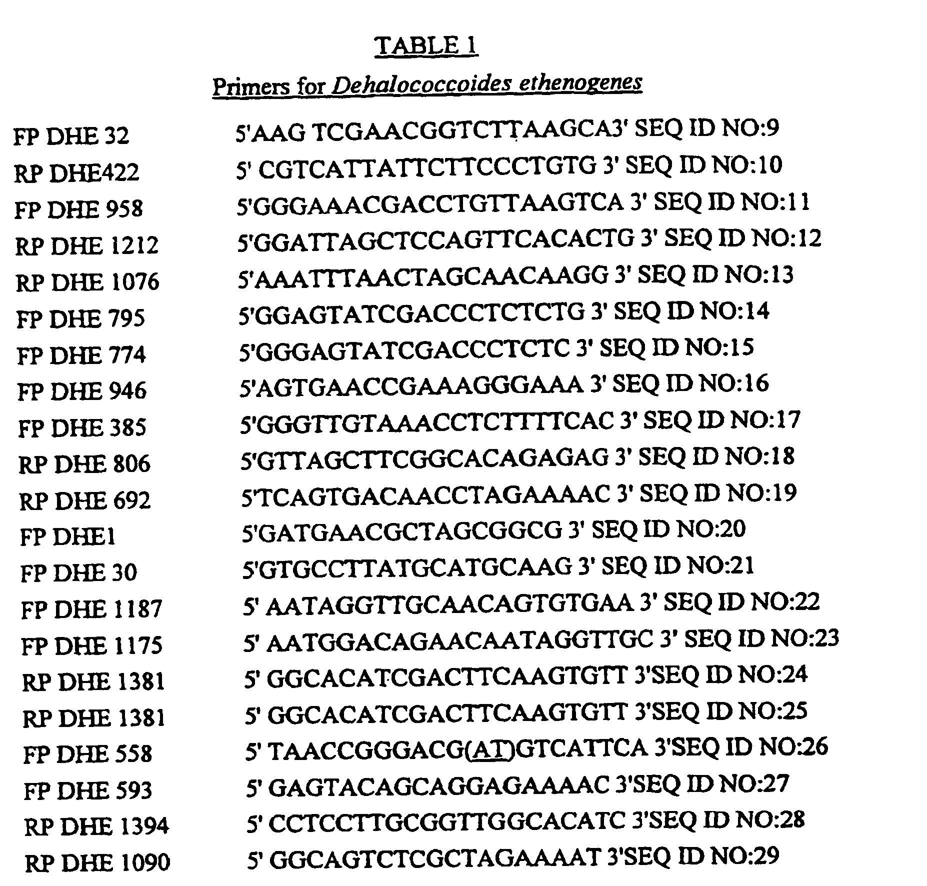

### EXAMPLE 3

### Using the Dehalococcoides Ethenogenes-Like Specific Primers to Detect These Organisms in Microcosms

Nucleic acids were extracted from the microcosm cultures by a bead mill homogenization procedure, FastDNA Spin Kit for Soil (Bio 101, Vista, CA), that was designed to isolate genomic DNA from all cell types. Approximately 10 mL of the microcosm culture was pelleted and resuspended in 500 ul of the culture media. The resuspended pellet was added to a 2.2 mL conical screw-cap tube containing 1.5 g of three differently sized glass and zirconia/silica beads (106 microns, 710-1180 microns). To the sample tubes, 978 ul of sodium phosphate buffer and 122 ul of MT buffer was added. The tubes were homogenized for 30 seconds at speed 5.5 on a Fast Prep bead mill homogenizer. A clear supernatant was obtained by centrifuging the samples at 14,000 x g for 30 seconds. The supernatant was transferred to a clean microcentrifuge tube and 250 ul of PPS reagent was added and mixed. The resulting precipitate was pelleted through centrifugation at 14,000 x g for 5 minutes. The supernatant was transferred to a new microcentrifuge tube and 1 mL of binding matrix was added. The samples were placed on a rotator for 2 minutes and then sat on the benchtop for 3 minutes to allow the settling of the silica matrix. Between 500-700 ul of the supernatant was removed and discarded. The remaining supernatant was used to resuspend the silica matrix and transferred to a spin filter. The spin filter was centrifuged for 1 minute at 14,000 x g and the flow-through decanted. The silica matrix was washed with 500 ul of SEWS-M buffer and centrifuged for 1 minute at 16,000 x g. The flow through was discarded and any residual buffer in the matrix was removed by a 2 minute centrifugation at 14,000 x g. The spin filter was placed in a catch tube and air dried for 5 minutes in a biological hood. The genomic DNA was eluted by adding 60 ul of sterile, deionized water, mixing the matrix and the water together with a pipet tip, and centrifuging for 1 minute at 14,000 x g.

The 16S rRNA gene for *Dehalococcoides ethenogenes* was detected by PCR amplification and gel electrophoresis. The 16S sequences were amplified using *Dehalococcoides ethenogenes* specific 16S rDNA primers shown in Table 1. All PCR amplifications were performed using the GeneAmp PCR kit with Taq DNA polymerase (PE Applied Biosystems, Branchburg, NJ) in a Perkin Elmer 9600 thermal cycler Amplification reactions contained 10 mM Tris-HCl (pH 8.3), 50 mM KCl, 1.5 mM MgCl₂, 10 µM each deoxynucleoside triphosphate, 20 pmol each primer, 2.5 U of Taq polymerase, and 1 µL of the genomic extraction diluted 1:10 in a final reaction volume of 50 µL. The PCR conditions were as follows: 2 minutes of denaturation at 95 °C, followed by 30 cycles of 30 seconds at 94 °C, 30 seconds at 55 °C, 30 seconds at 72 °C. 8 µL of the PCR product was visualized on a 2% agarose gel (SeaKem GTG, FMC BioProducts, Rockland, ME) stained with ethidium bromide.

A direct detection protocol used 1 µL of the microcosm culture was directly added to the PCR as described previously.

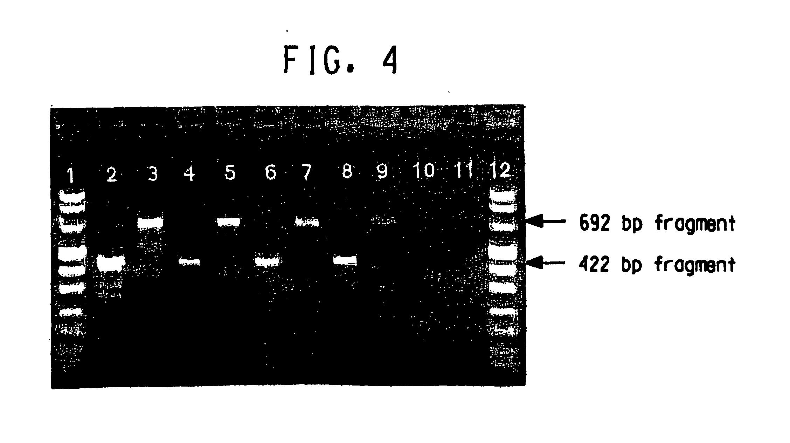

After the *Dehalococcoides ethenogenes*-like sequences were detected in the microcosm developed from contaminated soil, FP DHE 1 (SEQ ID NO:20), RP DHE 1330 (SEQ ID NO:12) were used to amplify a 1212 bp (or 1221 bp) fragment, which was cloned (using the PCR dA/T-Cloning System, Invitrogen, Inc., CA) and sequenced (using Model 377 DNA Sequencer kit and system, Applied Biosystems, Perkin-Elmer, Foster City, CA). The sequence was assembled using the Seqman II program (DNAstar, Inc., Madison, WI). The 16S rDNA sequence contig formed was compared to 16S rDNA sequences obtained from microcosms developed from contaminated soils from other sites and the comparison is shown in Figure 4.

Figure 4 shows a gel of amplification products generated from PCR amplification of various *Dehalococcoides ethenogenes* isolated from a number of industrial sites contaminated with either PCE or TCE. All amplifications were carried out using primers SEQ ID NOs:17 paired with 19, and SEQ ID NOs: 18 paired with 20. Lanes 1 and 12 carry the molecular weight markers. Lanes 2 and 3 are the PCR products generated from organisms isolated from soil containing PCE. Lanes 4, 5, 6, 7, 8 and 9 are the PCR products from organisms isolated from soil containing TCE. Lanes 10 and 11 contain negative PCR controls. As can be seen by the data all samples were detectable by the primers used.

The contiguous sequences from each site was unique, having 96 to 99% similarity to each other. The differences in the sequence are annotated in Table 2. A major difference exists in the consensus sequence that were obtained from all strains (CS) detected at contaminated sites and the reference sequence represented by the published sequence from strain DHE-195 strain (Table 2). At DHE (CS) positions 1088-1096 (*E. coli* coordinates E1146-E1156) there exists a nine base deletion. The sequence in CS strains reads ATTTTCTAGCGAGACTG (SEQ ID NO:31); in the DHE-195 strain it reads ATTTTCTAGCGAGACTAGCGAGACTG (SEQ ID NO:32) (the double underlined sequence is the sequence deleted in the CS strain sequences. Differences in sequence were found at six other base positions as shown below in Table 2.

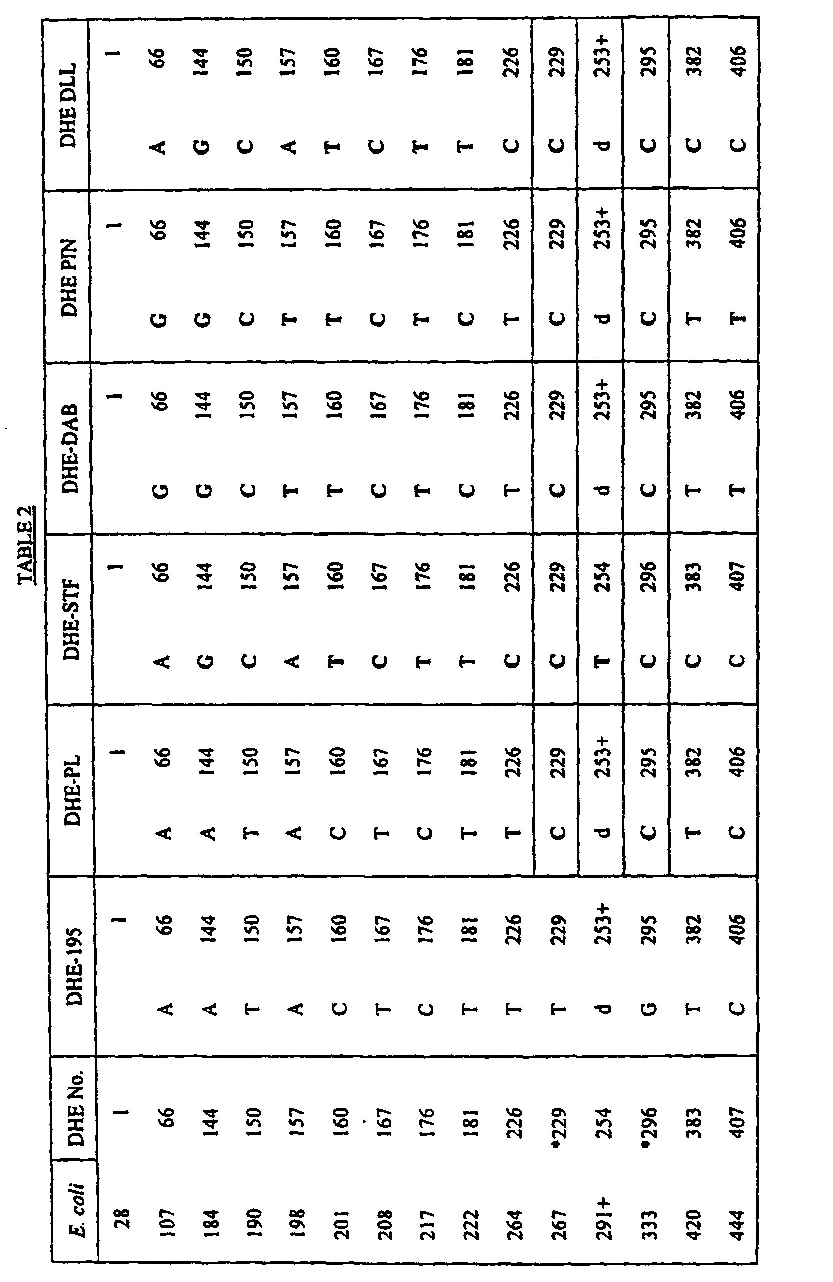

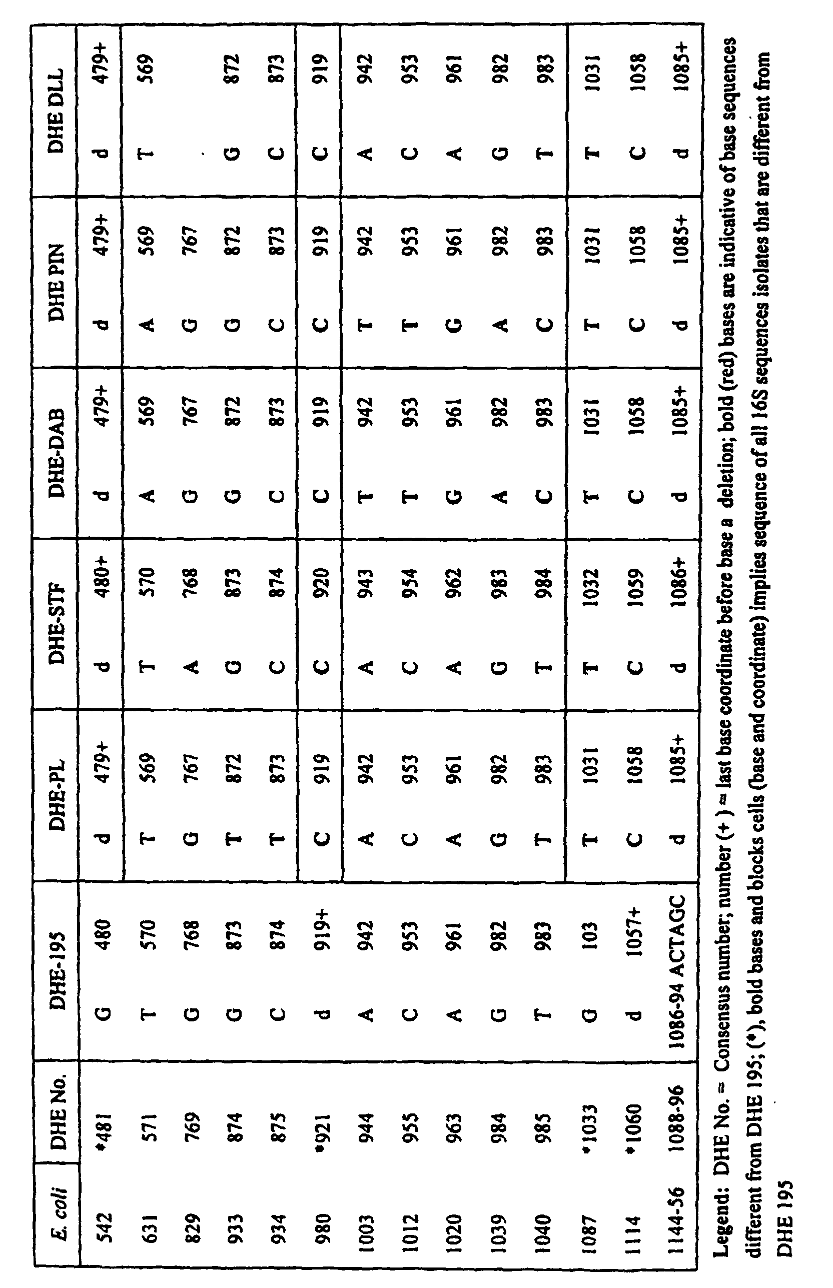

## Claims

1. An isolated 16S rDNA sequence indicative of a dechlorinating bacterial strain selected from the group consisting of:
(a) SEQ ID NO: 8 and SEQ ID NO: 30; and
(b) an isolated nucleic acid molecule that is completely complementary to (a).

2. An isolated bacterial strain comprising any one of the sequences of claim 1, wherein said strain has the ability to dechlorinate chlorinated compounds.

3. A method for identifying a dechlorinating bacterial strain comprising:
(i) extracting genomic DNA from a cell suspected of being able to dechlorinate chlorinated compounds;
(ii) probing the extracted genomic DNA with a probe derived from any one of the sequences of claim 1 under suitable hybridization conditions;
wherein the identification of a hybridizable nucleic acid fragment confirms the presence of a bacteria capable of dechlorinating chlorinated compounds.

4. A method for identifying a dechlorinating bacterial strain comprising:
(i) extracting genomic DNA from a cell suspected of being able to dechlorinate chlorinated compounds; and
(ii) amplifying the extracted genomic DNA with at least one oligonucleotide primer corresponding to a portion of any one of the sequences of claim 1 such that amplification products are generated;
wherein the presence of amplification products confirms the presence of a bacteria capable of dechlorinating chlorinated compounds.

5. A method for the dechlorination of chlorinated compounds comprising contacting a chlorinated compound with the isolated bacterial strain of claim 2 under conditions for the dechlorination to occur.

6. A method according to claim 5 wherein said dechlorinating compound is selected from the group consisting of carbontetrachloride, tetrachloroethene, chloroform, dichloromethane, trichloroethene, dichloroethylene, vinyl chloride and chloroaromatics.

## Patentansprüche

1. Isolierte 16S-rDNA-Sequenz, die auf einen dechlorierenden Bakterienstamm hindeutet, ausgewählt aus der Gruppe, bestehend aus:
(a) SEQ ID NO: 8 und SEQ ID NO: 30; und
(b) einem isolierten Nukleinsäuremolekül, das vollkommen komplementär zu (a) ist.

2. Isolierter Bakterienstamm, umfassend jedwede eine der Sequenzen nach Anspruch 1, worin der Stamm die Fähigkeit zum Dechlorieren chlorierter Verbindungen aufweist.

3. Verfahren zur Identifikation eines dechlorierenden Bakterienstamms, umfassend:
(i) Extrahieren genomischer DNA aus einer Zelle, die vermutlich zum Dechlorieren chlorierter Verbindungen fähig ist;
(ii) Sondieren der extrahierten genomischen DNA mit einer Sonde, die sich von jedweder einen der Sequenzen nach Anspruch 1 unter geeigneten; Hybridisierungsbedingungen herleitet,
worin die Identifikation eines hybridisierbaren Nukleinsäurefragments die Anwesenheit eines Bakteriums bestätigt, das zum Dechlorieren chlorierter Verbindungen fähig ist.

4. Verfahren zur Identifikation eines dechlorierenden Bakterienstamms, umfassend:
(i) Extrahieren genomischer DNA aus einer Zelle, die vermutlich zum Dechlorieren chlorierter Verbindungen fähig ist; und
(ii) Amplifizieren der extrahierten genomischen DNA mit mindestens einem Oligonukleotid-Primer, der einem Anteil von jedweder einen der Sequenzen nach Anspruch 1 dergestalt entspricht, dass Amplifikationsprodukte gebildet werden;
worin die Anwesenheit von Amplifikationsprodukten die Anwesenheit eines Bakteriums bestätigt, das zum Dechlorieren chlorierter Verbindungen fähig ist.

5. Verfahren zum Dechlorieren chlorierter Verbindungen, umfassend das Kontaktieren einer chlorierten Verbindung mit dem isolierten Bakterienstamm nach Anspruch 2 unter Bedingungen, unter denen die Dechlorierung auftreten kann.

6. Verfahren nach Anspruch 5, worin die dechlorierende Verbindung aus der Gruppe ausgewählt ist, bestehend aus Kohlenstofftetrachlorid, Tetrachlorethen, Chloroform, Dichlormethan, Trichlorethen, Dichlorethylen, Vinylchlorid und Chloraromaten.

## Revendications

1. Séquence d'ADNr 16S isolée indiquant une souche bactérienne de déchloration sélectionnée dans le groupe consistant en:
(a) SEQ ID NO: 8 et SEQ ID NO: 30; et
(b) une molécule d'acides nucléiques isolée qui est complètement complémentaire à (a).

2. Souche bactérienne isolée comprenant l'une quelconque des séquences selon la revendication 1, dans laquelle ladite souche a la capacité de déchlorer des composés chlorés.

3. Procédé pour identifier une souche bactérienne de déchloration comprenant:
(i) l'extraction d'ADN génomique à partir d'une cellule suspectée d'être capable de déchlorer des composés chlorés;
(ii) l'examen de l'ADN génomique extrait avec une sonde dérivée de l'une quelconque des séquences selon la revendication 1 dans des conditions d'hybridation adaptées;
dans lequel l'identification d'un fragment d'acides nucléiques pouvant être hybridé confirme la présence d'une bactérie capable de déchlorer des composés chlorés.

4. Procédé pour identifier une souche bactérienne de déchloration comprenant:
(i) l'extraction d'ADN génomique à partir d'une cellule suspectée d'être capable de déchlorer des composés chlorés; et
(ii) l'amplification de l'ADN génomique extrait avec au moins une amorce oligonucléotidique correspondant à une partie de l'une quelconque des séquences selon la revendication 1 qui fait que des produits d'amplification sont générés;
dans lequel la présence de produits d'amplification confirme la présence d'une bactérie capable de déchlorer des composés chlorés.

5. Procédé pour la déchloration de composés chlorés comprenant la mise en contact d'un composé chloré avec la souche bactérienne isolée selon la revendication 2 dans des conditions pour que la déchloration se produise.

6. Procédé selon la revendication 5 dans lequel ledit composé de déchloration est sélectionné dans le groupe constitué de tétrachlorure de carbone, tétrachloroéthène, chloroforme, dichlorométhane, trichloroéthène, dichloroéthylène, chlorure de vinyle et chloroaromatiques.
